(19) **Europäisches Patentamt** / **European Patent Office** / **Office européen des brevets**

(11) **EP 3 625 308 B1**

(12) **FASCICULE DE BREVET EUROPEEN**

(45) Date de publication et mention de la délivrance du brevet:
**15.09.2021 Bulletin 2021/37**

(21) Numéro de dépôt: **18724572.5**

(22) Date de dépôt: **18.05.2018**

(51) Int Cl.:
**C09K 11/02** *(2006.01)*        **C09K 11/82** *(2006.01)*

(86) Numéro de dépôt international:
**PCT/EP2018/063196**

(87) Numéro de publication internationale:
**WO 2018/211109 (22.11.2018 Gazette 2018/47)**

(54) **PARTICULES LUMINESCENTES A BASE DE TERRES RARES ET LEUR UTILISATION A TITRE D'AGENT DE DIAGNOSTIC**

LUMINESZENTE PARTIKEL AUF BASIS VON SELTENERDELEMENTEN UND VERWENDUNG DAVON ALS DIAGNOSTIKUM

LUMINESCENT PARTICLES BASED ON RARE EARTH ELEMENTS AND USE THEREOF AS A DIAGNOSTIC AGENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.05.2017 FR 1754416**

(43) Date de publication de la demande:
**25.03.2020 Bulletin 2020/13**

(73) Titulaires:
• **Ecole Polytechnique**
  **91120 Palaiseau (FR)**
• **Centre National de la Recherche Scientifique**
  **75794 Paris Cedex 16 (FR)**

(72) Inventeurs:
• **GACOIN, Thierry**
  **91440 Bures-Sur-Yvette (FR)**
• **RICHLY, Maximilian**
  **75006 Paris (FR)**
• **PREIRA, Pascal**
  **91600 Savigny-Sur-Orge (FR)**
• **ALEXANDROU, Antigoni**
  **91120 Palaiseau (FR)**
• **BOUZIGUES, Cédric**
  **75116 Paris (FR)**
• **MOHAMMEDI, Rabei**
  **91190 Gif-Sur-Yvette (FR)**

(74) Mandataire: **Nony**
  **11 rue Saint-Georges**
  **75009 Paris (FR)**

(56) Documents cités:
**WO-A1-2016/079069**

• **DOMITILLE GIAUME ET AL: "Functionalized Luminescent Oxide Nanoparticles as Biological Probes", MRS PROCEEDINGS, vol. 942, 1 janvier 2006 (2006-01-01), XP055445327, DOI: 10.1557/PROC-0942-W12-05**
• **JUAN WANG ET AL: "CTAB-assisted hydrothermal synthesis of YVO:Eupowders in a wide pH range", SOLID STATE SCIENCES, ELSEVIER, PARIS, FR, vol. 14, no. 1, 21 octobre 2011 (2011-10-21), pages 191-196, XP028339289, ISSN: 1293-2558, DOI: 10.1016/J.SOLIDSTATESCIENCES.2011.10.019 [extrait le 2011-10-29]**

Remarques:
Le dossier contient des informations techniques présentées postérieurement au dépôt de la demande et ne figurant pas dans le présent fascicule.

## Description

La présente invention concerne le domaine des sondes pour la détection et/ou la quantification de substances d'intérêt dans des échantillons biologiques. Elle a plus particulièrement pour objet de nouvelles particules inorganiques luminescentes, leur procédé de préparation et leur utilisation pour détecter et/ou doser la présence de protéines, d'anticorps, d'ADN, ARN et autres composés dans un échantillon biologique.

La détection et/ou la quantification des concentrations de biomarqueurs, d'anticorps ou d'ADN et ARN dans des échantillons biologiques (sang, sérum, salive, urine, liquide cérébro-spinal, etc.) sont indispensables pour le diagnostic médical.

Dans le domaine des diagnostics *in vitro* ou *ex vivo,* de l'analyse médicale et de la bioanalyse, un certain nombre de méthodes ont été proposées pour détecter et/ou doser la présence de substances spécifiques.

Ces méthodes reposent, d'une manière générale, sur l'utilisation d'une sonde mise en œuvre pour détecter et/ou quantifier une concentration en solution. Ces sondes sont couplées à des molécules, ADN ou protéines qui, directement ou *via* une série de réactions, vont se fixer à l'espèce moléculaire à analyser. Ensuite, les sondes qui ont réagi peuvent être détectées au moyen d'une ou plusieurs méthodes basées par exemple sur leur luminescence, leur absorbance, leur réactivité chimique, leur radioactivité, etc..

Les essais biochimiques les plus couramment mis en œuvre sont des essais d'immuno-absorption enzymatique (en langue anglaise « Enzyme-Linked Immuno Assay » ou ELISA), qui reposent généralement sur l'utilisation de la peroxydase de raifort comme enzyme pour provoquer une réaction avec un substrat et quantifier la réaction chimique ayant lieu par mesure de l'absorbance du produit de la réaction dans la solution. Le choix du composé moléculaire auquel la sonde est couplée est déterminant pour l'efficacité de ces sondes. Plus précisément, l'efficacité de ces méthodes dépend de l'affinité spécifique du composé avec la substance cible. A titre d'exemples, les publications référencées [1] et [2] détaillent les caractéristiques de ces mécanismes.

Les sondes luminescentes mènent généralement à une détection plus sensible que les sondes détectées par leur absorbance car, dans le premier cas, les mesures de l'intensité lumineuse se font sur fond noir, tandis que, dans le deuxième, il s'agit de mesurer une variation de l'intensité lumineuse (mesures sur fond clair). Les sondes luminescentes actuellement disponibles présentent plusieurs désavantages qui ne permettent pas d'exploiter pleinement leur potentiel en tant que sondes de diagnostic. Parmi ces inconvénients, on peut citer par exemple le phénomène de photoblanchiment dans le cas de fluorophores organiques qui, suite à des modifications structurales irréversibles, se traduit pas une disparition de la fluorescence, ou encore le phénomène de clignotement de l'émission pour les nanocristaux de semiconducteurs, ou « quantum dots », les sondes cessant alors périodiquement d'émettre et étant par conséquent inadaptées pour produire un signal constant. D'autres inconvénients résultent par exemple de la largeur du spectre d'émission des sondes luminescentes. De fait, un spectre d'émission trop large rend difficile le filtrage du signal de fond éventuellement présent, et a une incidence sur la qualité du signal et, en particulier, sur le rapport signal à bruit. Il convient également de prendre en compte, en plus des facteurs optiques qui contribuent à l'efficacité de la sonde dans un test biologique, le caractère pratique et la facilité d'utilisation de la sonde. Ainsi, certaines particules, comme c'est le cas des nanocristaux de semi-conducteurs, perdent leurs caractéristiques de luminescence après congélation, ce qui représente un inconvénient pour le stockage des agents bio-conjugués. La facilité de couplage des sondes au composé moléculaire permettant de cibler les molécules souhaitées est également un aspect à prendre en considération pour le choix de la sonde adéquate. Ainsi, un certain nombre de particules, dont les nanocristaux de semi-conducteurs sont synthétisées dans des solvants organiques. Il en découle qu'une utilisation pour des applications biologiques nécessite des étapes supplémentaires de préparation de la surface pour réaliser une dispersion dans l'eau de ces particules, processus qui peut être complexe à mettre en œuvre et peu stable dans le temps.

Par ailleurs, les propriétés colloïdales des particules/sondes sont déterminantes pour la conduite des essais biologiques. De fait, des solutions de bonne stabilité colloïdale sont à même de fournir des milieux pour les essais de bonne homogénéité, et dès lors une meilleure reproductibilité au niveau des résultats de ces essais.

Enfin, la complexité et le coût sont des aspects importants dans le choix des sondes pour le diagnostic. Par exemple, des nanoparticules d'or, et leurs propriétés en termes de résonance plasmonique de surface, ont été proposées comme sondes de diagnostic, mais n'ont pas percé comme sondes pour le diagnostic *in vitro,* possiblement à cause de la complexité de la méthode de détection [3], ou éventuellement d'un coût élevé. Quant aux cristaux de semiconducteurs, ils sont synthétisés dans des solvants organiques et requièrent des procédures de dispersion en milieu aqueux, ce qui rend leur synthèse complexe et ainsi coûteuse. Leur fonctionnalisation avec des groupements chimiques permettant le couplage aux composés moléculaires de reconnaissance des molécules cibles repose en outre sur des liaisons chimiques faibles, ce qui limite par conséquent leur stabilité, ce qui est préjudiciable pour la reproductibilité de tests de détection.

A ce titre, les nanoparticules dopées par des ions de terres rares, telles que des nanoparticules de type $YVO_4$:Eu, s'avèrent particulièrement avantageuses pour une mise en œuvre à titre de sondes pour la détection/quantification de biomolécules.

Des nanoparticules à base de vanadate d'yttrium dopé par des terres rares ont par exemple été décrites de façon détaillée par Riwotzki *et al.* [4] et Huignard *et al.* [5].

Ces nanoparticules sont particulièrement avantageuses au regard de leur excellente photostabilité, qui autorise l'acquisition d'un signal constant et prolongé, et une absence de phénomène de clignotement de l'émission. De plus, elles présentent un spectre d'émission étroit et un grand décalage de Stokes de l'émission. Ces deux caractéristiques permettent de filtrer efficacement le signal d'émission des nanoparticules en s'affranchissant des signaux parasites pour obtenir d'excellents rapports signal à bruit. Ces nanoparticules contiennent également plusieurs dizaines de milliers d'ions pouvant être excités et responsables de la luminescence. L'augmentation de l'intensité d'excitation induit ainsi une augmentation linéaire de la luminosité des particules, la saturation de l'émission n'étant atteinte que pour des intensités impraticables.

Enfin, ces nanoparticules ne perdent pas leur luminescence après congélation.

La synthèse en milieu solvant organique de nanoparticules luminescentes dopées par des ions de terres rares a par exemple été proposée dans le document EP 1 232 226 ou dans les publications [6], [7] et [8].

Il a également déjà été proposé la synthèse de ces nanoparticules luminescentes en milieu aqueux, par exemple par addition goutte-à-goutte de solutions de nitrates d'yttrium et d'europium à une solution d'orthovanadate ($Na_3VO_4$) ([9], [10], [11], [12] et [13]).

Par ailleurs, les publications [14]-[19] proposent des méthodes de synthèse par voie hydrothermale de nano-particules d'$YVO_4$ dopées par des ions de terres rares qui mettent en œuvre à titre de précurseur du métavanate d'ammonium ou de l'orthovanadate.

On peut encore citer le document US 8 337 804 qui propose une méthode de préparation de nanoparticules à base d'ions orthovanadate $VO_4^{3-}$, par exemple de $YVO_4$ :Eu, en mettant en œuvre un milieu de réaction contenant de l'eau et au moins un polyol.

Quant au document EP 1 282 824, il décrit la mise en œuvre de nanoparticules luminescentes inorganiques, modifiées en surface, à titre de sondes pour détecter une substance biologique ou autre substance organique.

La publication Giaume et al., Mater. Res. Soc. Symp. Proc. Vol. 942, 2006, décrit la synthèse d'une solution colloïdale de nanoparticules de type $Y_{1-x}Eu_xVO_4$ (0<x<1) par addition d'une solution aqueuse comprenant des nitrates d'yttrium et d'europium, à une solution aqueuse d'orthovanadate de sodium. Le pH de la solution d'orthovanadate de sodium est ajusté à une valeur comprise entre 12,5 et 13 par addition d'hydroxyde de tétraméthylammonium, de manière à obtenir un pH final d'environ 9. En vue d'améliorer la stabilité colloïdale, Giaume *et al.* proposent la mise en œuvre d'une étape de traitement de surface des nanoparticules, opérée à l'issue de leur synthèse, par adsorption d'ions silicate chargés négativement au niveau des nanoparticules.

De même, le document WO 2016/079069 décrit un procédé de préparation par voie sol-gel d'une solution colloïdale de nanoparticules de type $Y_xEu_{1-x}VO_4$, mettant en œuvre l'ajout de solutions de nitrate d'europium et d'yttrium à une solution d'orthovanadate de sodium, et un ajustement du pH par ajout d'une base organique ou inorganique tel que l'hydroxyde de tétraméthylammonium.

Les nanoparticules luminescentes, obtenues par les voies de synthèse connues précitées, et mises en œuvre en milieu aqueux, sont toutefois sujettes à des phénomènes de floculation, et ne permettent pas de conduire à des suspensions colloïdales stables. Dès lors, la quantité de nanoparticules en suspension est instable au cours du temps, et ne peut être déterminée de manière précise.

Cette incertitude au niveau de la teneur en particules luminescentes dans les suspensions colloïdales mises en œuvre est hautement préjudiciable lors de leur utilisation à titre de sondes dans des méthodes de diagnostic, puisqu'elle vient impacter la reproductibilité des résultats obtenus, par exemple lors de la détection/quantification des concentrations en biomolécules de l'échantillon testé.

En conséquence, les particules luminescentes dopées par des ions de terres rares obtenues par les méthodes de l'état de l'art ne permettent pas de donner entière satisfaction pour une mise en œuvre à titre de sondes de diagnostic.

La présente invention vise précisément à proposer de nouvelles particules luminescentes et une nouvelle voie pour leur synthèse, permettant de pallier les inconvénients précités.

En particulier, l'invention propose un moyen d'accéder à des nanoparticules luminescentes présentant une stabilité colloïdale améliorée en milieu aqueux, et avantageusement avec une meilleure reproductibilité de leur synthèse et des étapes ultérieures de fonctionnalisation et de couplage à un agent moléculaire de ciblage.

Ainsi, l'invention concerne, selon un premier de ses aspects, une particule luminescente comprenant une nanoparticule de formule :

$$A_{1-x}Ln_xVO_{4(1-y)}(PO_4)_y \qquad (I)$$

dans laquelle :

- A est choisi parmi l'yttrium (Y), le gadolinium (Gd), le lanthane (La) et leurs mélanges;

- Ln est choisi parmi l'europium (Eu), le dysprosium (Dy), le samarium (Sm), le néodyme (Nd), l'erbium (Er), l'ytterbium (Yb) et leurs mélanges ;
- 0<x<1, en particulier 0,2≤x≤0,6 et plus particulièrement x vaut 0,4 ; et
- 0≤y<1 ;

caractérisée en ce que la nanoparticule présente en surface des cations tétraalkylammonium en une quantité telle que ladite nanoparticule présente un potentiel zêta, noté $\zeta$, inférieur ou égal à -28 mV, dans un milieu aqueux de pH ≥ 5, en particulier de pH ≥ 5,5, et de conductivité ionique strictement inférieure à 100 $\mu$S.cm$^{-1}$.

Selon un autre de ses aspects, l'invention concerne un procédé de préparation de telles particules, mettant en œuvre une réaction de co-précipitation, en milieux aqueux, à partir de précurseurs des éléments A et Ln et en présence d'ions orthovanadate (VO$_4$$^{3-}$) et éventuellement phosphate (PO$_4$$^{3-}$); ladite réaction étant opérée en présence d'une quantité efficace de cations tétraalkylammonium telle que ladite nanoparticule présente un potentiel zêta, noté $\zeta$, inférieur ou égal à -28 mV, dans un milieu aqueux de pH ≥ 5, en particulier de pH ≥ 5,5, et de conductivité ionique strictement inférieure à 100 $\mu$S.cm$^{-1}$.

De préférence, ladite nanoparticule présente un potentiel zêta $\zeta$, en milieu aqueux à pH ≥ 6,5, en particulier à pH ≥ 7 et notamment à pH ≥ 8, inférieur ou égal à -30 mV.

Les particules luminescentes selon l'invention, et leur voie de synthèse, s'avèrent avantageuses à plusieurs titres.

Cette nouvelle voie de synthèse repose sur la mise en œuvre de contre-ions volumineux de type tétraalkylammonium.

Les inventeurs ont ainsi découvert que la mise en œuvre de ces contre-ions volumineux dans la synthèse des nanoparticules à base d'ions de terres rares assure une stabilité colloïdale améliorée des particules en solution, et prévient notamment les phénomènes de floculation des particules.

Comme évoqué précédemment, la stabilité des particules en solution est particulièrement déterminante pour répondre aux exigences en termes de reproductibilité pour la mise en œuvre de ces particules à titre de sondes pour le diagnostic *in vitro* ou *ex vivo*.

Sans vouloir être liée par la théorie, cette amélioration en termes de stabilisation, résultant de la mise en œuvre de contre-ions tétraalkylammonium, comparativement par exemple à la mise en œuvre d'ions sodium, est liée à la différence du potentiel zêta des particules.

Le « potentiel zêta », noté $\zeta$, peut être défini comme la différence de potentiel existant entre le sein de la solution, et le plan de cisaillement de la particule. Il est représentatif de la stabilité d'une suspension. Le plan de cisaillement (ou rayon hydrodynamique) correspond à une sphère imaginaire autour de la particule dans laquelle le solvant bouge avec la particule lorsque les particules se déplacent dans la solution. Le potentiel zêta peut être déterminé par des méthodes connues de l'homme du métier, par exemple par déplacement de la particule avec sa couche de solubilisation dans un champ électrique, comme détaillé dans la suite du texte.

De fait, comme illustré dans les exemples qui suivent, la mise en œuvre des contre-ions tétraalkylammonium induit un potentiel zêta négatif de la particule de valeur absolue accrue, comparativement par exemple au cas de la mise en œuvre de contre-ions sodium. Ce potentiel zêta négatif des nanoparticules, inférieur ou égal à -28 mV, en particulier inférieur ou égal à -30 mV, en milieu aqueux à pH ≥ 5, en particulier à pH ≥ 6,5, accroît les phénomènes de répulsion électrostatique des nanoparticules en solution aqueuse les unes par rapport aux autres, ce qui permet ainsi de supprimer les phénomènes de floculation. Il est en effet connu de façon empirique de l'homme du métier qu'un potentiel zêta de valeur absolue élevé, en particulier supérieure à 28 mV, permet en général de supprimer les effets de floculation dans des milieux à faible force ionique.

Ainsi, comme détaillé dans la suite du texte, les nanoparticules de l'invention, présentant en surface des cations tétraalkylammonium, possèdent un potentiel zêta, noté $\zeta$, en milieu aqueux à pH ≥ 5, inférieur ou égal à -28 mV. En particulier, les nanoparticules de l'invention, présentant en surface des cations tétraalkylammonium, possèdent en milieu aqueux à pH ≥ 6,5, un potentiel zêta inférieur ou égal à -30 mV.

Il est entendu que les mesures de potentiel zêta sont effectuées après purification de la suspension aqueuse des particules, et donc pour une suspension aqueuse présentant une conductivité ionique strictement inférieure à 100 $\mu$S.cm$^{-1}$.

La conductivité ionique de la suspension, permettant une appréciation du taux d'ions présents dans ladite suspension, peut être mesurée, à une température ambiante (25°C), par tout conductimètre connu.

L'invention concerne ainsi, selon un autre de ses aspects, une suspension aqueuse colloïdale comprenant des particules luminescentes selon l'invention.

Une suspension selon l'invention présente ainsi d'excellentes propriétés de stabilité colloïdale.

Par ailleurs, en termes de synthèse, les particules de l'invention sont synthétisées directement en milieu aqueux, et le protocole de synthèse requiert uniquement une réaction de co-précipitation. La préparation des nanoparticules de l'invention permet ainsi avantageusement de s'affranchir d'étapes d'élimination de solvants organiques et de transfert des nanoparticules dans d'autres solvants. Egalement, la synthèse des nanoparticules selon l'invention peut être avantageusement opérée à température ambiante.

Egalement, comme détaillé dans la suite du texte, les ions orthovanadate ($VO_4^{3-}$) mis en œuvre dans la réaction de co-précipitation pour former les nanoparticules de l'invention peuvent être avantageusement générés *in situ* à partir d'un sel de métavanadate, par exemple du métavanadate d'ammonium ($NH_4VO_3$) ou de sodium ($NaVO_3$), avec ajout de deux équivalents de base forte, avantageusement des hydroxydes de tétraalkylammonium.

L'utilisation du métavanadate à titre de précurseur permet avantageusement d'introduire des contre-ions volumineux de type tétraalkylammonium comme contre-ions de la base forte utilisée. Ainsi, la méthode de synthèse des particules selon l'invention présente une très bonne stabilité colloïdale.

Enfin, comme détaillé par la suite, les particules de l'invention peuvent être aisément couplées à des agents de ciblage à des fins de diagnostic, *in vivo, ex vivo* ou *in vitro, via* des réactions de fonctionnalisation/couplage variées. Les nanoparticules synthétisées selon l'invention trouvent ainsi une application particulièrement avantageuse en tant que sondes permettant la détection et/ou la quantification, *ex vivo* ou *in vitro,* dans des échantillons biologiques, avec une excellente reproductibilité.

L'invention concerne ainsi, selon un autre de ses aspects, l'utilisation de particules selon l'invention, en particulier couplées à au moins un agent de ciblage, à titre d'agent de diagnostic, en particulier à titre d'agent de diagnostic *in vitro* ou *ex vivo.*

Selon encore un autre de ses aspects, elle concerne un kit de diagnostic, en particulier *in vitro* ou *ex vivo,* autrement dit un kit de détection et/ou de quantification *in vitro* ou *ex vivo* d'une substance d'intérêt biologique ou chimique dans un échantillon, comprenant au moins des particules luminescentes selon l'invention, en particulier sous forme d'une suspension aqueuse colloïdale.

Dans la suite du texte, on désignera plus simplement sous l'appellation « nanoparticule selon l'invention », la nanoparticule répondant à la formule (I) précitée à la surface de laquelle sont localisés des cations tétraalkylammonium.

On emploie par ailleurs l'expression « nanoparticule fonctionnalisée » pour désigner la nanoparticule selon l'invention fonctionnalisée en outre en surface par un ou plusieurs groupements tels que par exemple par du citrate, du polyacide acrylique (PAA), des fonctions aldéhyde, des fonctions acide carboxylique (COOH), des fonctions amines, etc..

Enfin, l'expression « nanoparticule couplée » sera employée pour désigner la nanoparticule de l'invention à l'issue de son couplage, après fonctionnalisation de surface, avec un ou plusieurs agents de ciblage, tels que des protéines, anticorps, ADN, ARN, aptamère, etc., comme détaillé dans la suite du texte.

D'autres caractéristiques, variantes et avantages des particules selon l'invention, de leur procédé de synthèse et de leur mise en œuvre à titre de sondes pour le diagnostic ressortiront mieux à la lecture de la description, des exemples et des figures qui suivent, donnés à titre illustratif et non limitatif de l'invention.

Dans la suite du texte, les expressions « compris entre ... et ... », « allant de . à ... » et « variant de ... à ... » sont équivalentes et entendent signifier que les bornes sont incluses, sauf mention contraire.

Sauf indication contraire, l'expression « comprenant un(e) » doit être comprise comme « comportant au moins un(e) ».

## PARTICULES LUMINESCENTES

Comme précisé précédemment, les particules luminescentes de l'invention comprennent, voire sont formées, d'une nanoparticule de formule $A_{1-x}Ln_xVO_{4(1-y)}(PO_4)_y$ (I), dans laquelle A, x, Ln et y sont tels que définis précédemment, à la surface de laquelle sont présents des cations de type tétraalkylammonium.

L'immobilisation des cations de type tétraalkylammonium à la surface des nanoparticules de l'invention résulte plus particulièrement d'interactions électrostatiques entre les ions de surface de la nanoparticule de formule (I) chargés négativement ($O^{2-}$) et les contre-ions de type tétraalkylammonium chargés positivement.

Ainsi, les cations tétraalkylammonium sont associés directement à la nanoparticule *via* des interactions électrostatiques avec les ions de surface chargés négativement de la nanoparticule. En particulier, l'immobilisation des cations tétraalkylammonium à la surface de la nanoparticule ne met en œuvre aucun groupe « espaceur ».

Par cations « tétraalkylammonium », on entend plus particulièrement désigner des cations tétra($C_1$-$C_6$)alkylammonium, c'est-à-dire des cations de formule $NR_4^+$ avec R, identiques ou différents, représentant un groupe $C_1$-$C_6$-alkyl, en particulier $C_1$-$C_4$-alkyl.

De préférence, les cations « tétraalkylammonium » sont des cations tétra($C_1$-$C_3$)alkylammonium, c'est-à-dire des cations de formule $NR_4^+$ avec R, identiques ou différents, représentant un groupe $C_1$-$C_3$-alkyl.

Par « $C_1$-$C_6$-alkyl », on entend un groupe aliphatique saturé, linéaire ou ramifié, comprenant de 1 à 6 atomes de carbone. A titre d'exemples, on peut citer les groupes méthyle, éthyle, propyle, butyle, isopropyle, isobutyle, tertbutyle, etc.

Les cations tétraalkylammonium sont de préférence choisis parmi les cations tétraméthylammonium, tétraéthylammonium, tétrapropylammonium, tétrabutylammonium et leurs mélanges.

Selon un mode de réalisation particulier, les cations présents à la surface des nanoparticules de l'invention sont des cations tétraméthylammonium.

Comme indiqué précédemment, les cations tétraalkylammonium sont présents en surface des nanoparticules de l'invention en une quantité suffisante pour procurer le résultat souhaité en termes de stabilité colloïdale de la suspension aqueuse desdites particules synthétisées, autrement dit la valeur du potentiel zêta souhaitée.

Les cations tétraalkylammonium, présents à la surface des nanoparticules de l'invention, peuvent être présents à raison de 100 à 10000 cations tétraalkylammonium par nanoparticule.

Les nanoparticules de l'invention répondent ainsi plus particulièrement à la formule (I') suivante :

$$A_{1-x}Ln_xVO_{4(1-y)}(PO_4)_y \cdot (NR_4^+)_z \qquad (I')$$

dans laquelle :

- A est choisi parmi Y, Gd, La et leurs mélanges, de préférence A représente Y ;
- Ln est choisi parmi Eu, Dy, Sm, Nd, Er, Yb et leurs mélanges, de préférence Ln représente Eu ;
- $0<x<1$, en particulier $0{,}2 \leq x \leq 0{,}6$ et plus particulièrement x vaut 0,4 ;
- $0 \leq y < 1$ ;
- R, identiques ou différents, sont tels que définis précédemment, de préférence représentent un groupe $C_1$-$C_3$-alkyl, en particulier méthyle ; et
- z représente le nombre de cations tétraalkylammonium $NR_4^+$ localisés à la surface de ladite nanoparticule, en particulier z est compris entre 100 et 10000.

Selon un mode de réalisation particulier, la nanoparticule répond à la formule (I) ou (I') précitée dans laquelle y vaut 0.

Autrement dit, la nanoparticule de l'invention est de formule $A_{1-x}Ln_xVO_4$ (II), à la surface de laquelle sont localisés des cations tétraalkylamonium.

Ainsi, une nanoparticule de l'invention peut répondre plus particulièrement à la formule $A_{1-x}Ln_xVO_4 \cdot (NR_4^+)_z$ (II'), dans laquelle A, x, Ln, R et z sont tels que définis précédemment.

Selon un mode de réalisation particulier, A dans la formule (I), (I'), (II) ou (II') précitée représente l'yttrium (Y).

Selon un autre mode de réalisation particulier, Ln dans la formule (I), (I'), (II) ou (II') précitée représente Eu.

Ainsi, selon une variante de réalisation particulière, les particules de l'invention sont formées en tout ou partie d'une nanoparticule de formule $Y_{1-x}Eu_xVO_4$ (III), avec $0<x<1$, ladite nanoparticule présentant en surface des cations tétraalkylammonium.

Plus particulièrement, une nanoparticule de l'invention peut répondre plus particulièrement à la formule $Y_{1-x}Eu_xVO4 \cdot (NR_4^+)_z$ (III'), dans laquelle x, R et z sont tels que définis précédemment.

Selon un mode de réalisation particulier, le produit entre le taux de dopage, x, en ions Ln, en particulier en europium (Eu), et le rendement quantique de l'émission par la nanoparticule est maximisé.

En particulier, cela est utile lorsque l'on prévoit une excitation optique des ions Ln par excitation directe, *i.e.* en résonance avec les états électroniques de ces ions, et non par excitation de la matrice $AVO_{4(1-y)}(PO_4)_y$ et transfert d'énergie ultérieur vers ces ions.

Cette optimisation du produit entre le taux de dopage x en ions Ln et le rendement quantique peut être effectuée en utilisant un fort dopage en ions Ln, par exemple entre 0,2 et 0,6, et notamment de 0,4, sans pour autant diminuer le rendement quantique, en particulier en limitant les processus de transfert entre ions de dopage conduisant à une extinction de concentration. En particulier, afin de maintenir un rendement quantique élevé, il est nécessaire que la cristallinité de la particule ne soit pas parfaite. En effet, une excellente cristallinité favorise les processus de transfert entres ions de dopage, en particulier lorsque ceux-ci sont à proximité immédiate entre eux, comme cela est le cas pour des dopages élevés et, par conséquent, favorise les processus de désexcitation des ions par des processus non radiatifs liés au solvant. En particulier, un procédé de synthèse à température ambiante, ou tout au moins à température n'excédant pas 600°C, est favorable pour la cristallinité imparfaite requise pour ces nanoparticules.

La cristallinité des nanoparticules est considérée comme étant « imparfaite » lorsque la longueur de cohérence déterminée par le diffractogramme des rayons X dans au moins une direction cristallographique donnée est inférieure à 80 % de la taille de la particule dans cette direction-là telle que mesurée à partir des images de microscopie électronique à transmission. Différents types de cristallinité imparfaite peuvent être considérés : polycristallinité, défauts ou porosité.

A titre d'exemple, la nanoparticule peut être de formule $Y_{0{,}6}Eu_{0{,}4}VO_4$ à la surface sont localisés des cations tétraalkylammonium,. En particulier, elle peut répondre à la formule $Y_{0{,}6}Eu_{0{,}4}VO_4 \cdot (NR_4^+)_z$, z représentant le nombre de cations tétraalkylammonium.

Il est entendu que les différents modes de réalisation précités, notamment en ce qui concerne la nature de la nanoparticule photoluminescente et des cations tétraalkylammonium de surface, peuvent être combinés.

A titre d'exemple, une particule photoluminescente selon l'invention peut être formée d'une nanoparticule de formule $Y_{0,6}Eu_{0,4}VO_4$ à la surface de laquelle sont immobilisés des cations tétraméthylammonium.

Les nanoparticules de l'invention sont plus particulièrement de forme globalement ellipsoïdale allongée (« prolate » en langue anglaise).

La taille peut être mesurée par microscopie électronique à transmission. Les images de microscopie électronique à transmission permettent de déterminer la forme des nanoparticules (ellipsoïde allongée) et de déduire les dimensions moyennes des deux axes de l'ellipsoïde. On suppose en général que le troisième axe de l'ellipsoïde, non visible dans les images de transmission qui sont des projections 2D, est de longueur égale à l'axe de plus petite taille.

Les nanoparticules de l'invention, de forme ellipsoïde allongée, peuvent présenter une longueur du grand axe, notée *a*, comprise entre 20 et 60 nm ; et une longueur du petit axe, notée b, comprise entre 10 et 30 nm. En particulier, les nanoparticules de l'invention peuvent présenter une valeur moyenne de longueur du grand axe, *a,* de 40 nm et une valeur moyenne de longueur du petit axe, *b,* de 20 nm.

Comme évoqué précédemment, les particules luminescentes selon l'invention permettent d'accéder à des suspensions aqueuses présentant une très bonne stabilité colloïdale. A noter que cette stabilité est importante même dans des milieux de force ionique élevée comme c'est le cas dans le milieu de synthèse même avant purification (force ionique supérieure à 0,1 M). En particulier, une suspension aqueuse de particules selon l'invention présente peu, voire pas, de phénomène de floculation.

En particulier, les particules selon l'invention à la surface desquelles sont présents des cations tétraalkylammonium présentent un potentiel zêta, noté $\zeta$, inférieur ou égal à -28 mV, dans un milieu aqueux de pH $\geq$ 5, en particulier de pH $\geq$ 5,5, et présentant une conductivité ionique strictement inférieure à 100 $\mu$S.cm$^{-1}$.

Autrement dit, la valeur absolue du potentiel zêta, notée $|\zeta|$, des particules de l'invention, en milieu aqueux à pH $\geq$ 5, en particulier de pH $\geq$ 5,5, est supérieure à 28 mV.

Le « potentiel zêta » est un des éléments représentatifs de la stabilité d'une suspension. Il peut être par exemple directement mesuré à l'aide d'un équipement de type Zetasizer Nano ZS de la société Malvern. Cet équipement mesure à l'aide de dispositifs optiques les vitesses de déplacement des particules en fonction du champ électrique qui leur est appliqué.

Les nanoparticules de l'invention possèdent plus particulièrement un potentiel zêta inférieur ou égal à -28 mV, de préférence inférieur ou égal à -30 mV, dans un milieu aqueux de pH $\geq$ 6, en particulier de pH $\geq$ 6,5, et de conductivité ionique strictement inférieure à 100 $\mu$S.cm$^{-1}$.

En particulier, les nanoparticules de l'invention possèdent un potentiel zêta inférieur ou égal à -30 mV, dans un milieu aqueux de pH $\geq$ 6,5, en particulier de pH $\geq$ 7, notamment de pH $\geq$ 8, et de conductivité ionique strictement inférieure à 100 $\mu$S.cm$^{-1}$.

Les nanoparticules selon l'invention sont de nature majoritairement cristalline et polycristalline, en particulier de taille moyenne de cristallites, déduite par diffraction de rayons X, comme détaillé dans les exemples qui suivent, comprise entre 3 et 40 nm.

Selon un mode de réalisation particulier de l'invention, les nanoparticules selon l'invention sont couplées (ou greffées) à au moins un agent de ciblage, en vue notamment de leur utilisation comme sondes dans des méthodes de diagnostic, en particulier des méthodes de diagnostic *in vitro* ou *ex vivo,* pour la détection et/ou quantification d'espèces biologiques dans un échantillon biologique.

Autrement dit, les particules de l'invention peuvent comprendre au moins un agent de ciblage immobilisé à la surface de la nanoparticule.

Les agents de ciblage peuvent être de nature variée comme détaillé dans la suite du texte, en fonction de l'application recherchée pour les particules luminescentes de l'invention.

## PREPARATION DES PARTICULES

Comme évoqué précédemment, l'invention concerne encore, selon un autre de ses aspects, un procédé de préparation de particules luminescentes comprenant une nanoparticule de formule :

$$A_{1-x}Ln_xVO_{4(1-y)}(PO_4)_y \qquad (I)$$

dans laquelle :

- A est choisi parmi l'yttrium (Y), le gadolinium (Gd), le lanthane (La) et leurs mélanges, de préférence A représente Y ;
- Ln est choisi parmi l'europium (Eu), le dysprosium (Dy), le samarium (Sm), le néodyme (Nd), l'erbium (Er), l'ytterbium (Yb), et leurs mélanges, de préférence Ln représente Eu ;
- $0 < x < 1$ ; et
- $0 \leq y < 1$.

par réaction de co-précipitation, en milieu aqueux, à partir de précurseurs desdits éléments A et Ln, et en présence d'ions orthovanadate ($VO_4^{3-}$) et éventuellement d'ions phosphate ($PO_4^{3-}$); ladite réaction étant opérée en présence d'une quantité efficace de cations tétraalkylammonium telle que ladite nanoparticule présente un potentiel zêta, noté $\zeta$, inférieur ou égal à -28 mV, dans un milieu aqueux de pH $\geq$ 5, en particulier de pH $\geq$ 5,5, et de conductivité ionique strictement inférieure à 100 $\mu$S.cm$^{-1}$.

En particulier, l'invention concerne un procédé de préparation de particules luminescentes comprenant une nanoparticule de formule (I') :

$$A_{1-x}Ln_xVO_{4(1-y)}(PO_4)_y \cdot (NR_4^+)_z \qquad (I')$$

dans laquelle :

- A représente Y, Gd, La ou leurs mélanges, de préférence A représente Y ;
- Ln est choisi parmi Eu, Dy, Sm, Nd, Er, Yb et leurs mélanges, de préférence Ln représente Eu ;
- $0<x<1$ ;
- $0\leq y<1$ ;
- R, identiques ou différents, sont tels que définis précédemment, de préférence représentent un groupe $C_1$-$C_3$-alkyl ; et
- z représente le nombre de cations tétraalkylammonium $NR_4^+$ localisés à la surface de ladite nanoparticule.

Plus particulièrement, l'invention concerne un procédé de préparation de particules luminescentes comprenant une nanoparticule de formule (II), (II'), (III) ou (III') telle que définie précédemment.

Les précurseurs des éléments A et Ln peuvent se présenter, de manière classique, sous la forme de sels desdits éléments, par exemple de nitrates, chlorures, perchlorates ou acétates, en particulier de nitrates. Les précurseurs des éléments A et Ln, et leur quantité, sont bien entendu choisis de manière adéquate au regard de la nature de la nanoparticule souhaitée.

Par exemple, la synthèse de nanoparticules de formule $Y_{1-x}Eu_xVO_4$ (III) peut mettre en œuvre, à titre de composés précurseurs de l'yttrium et de l'europium, les nitrates d'yttrium ($Y(NO_3)_3$) et d'europium ($Eu(NO_3)_3$).

Selon une caractéristique essentielle du procédé de l'invention, la réaction de co-précipitation est réalisée en présence d'une quantité efficace de cations tétraalkylammonium.

Les cations tétraalkylammonium sont plus particulièrement tels que définis précédemment. Ils sont de préférence choisis parmi les cations tétraméthylammonium, tétraéthylammonium, tétrapropylammonium, tétrabutylammonium et leurs mélanges. Il s'agit de préférence de cations tétraméthylammonium.

Par « quantité efficace », on entend plus particulièrement signifier que les cations sont mis en œuvre en une quantité suffisante pour procurer le résultat souhaité en termes de stabilité colloïdale de la suspension aqueuse desdites particules synthétisées. Les cations tétraalkylammonium sont mis en œuvre en une quantité telle que les nanoparticules obtenues à l'issue du procédé de l'invention présentent un potentiel zêta tel qu'indiqué précédemment. Comme illustré en exemple 1 qui suit, les cations tétraalkylammonium, dans la réaction de co-précipitation selon l'invention, sont des contre-ions des ions orthovanadate ($VO_4^{3-}$), et éventuellement phosphate ($PO_4^{3-}$) (dans le cas où $y \neq 0$).

Selon un mode de réalisation particulièrement préféré, les ions orthovanadate ($VO_4^{3-}$) sont générés *in situ* à partir d'un sel de métavanadate, de préférence du métavanadate d'ammonium ($NH_4VO_3$).

Les ions orthovanadate peuvent être plus particulièrement formés *in situ* par réaction dudit sel de métavanadate avec une base (plus précisément avec deux équivalents de base forte).

En particulier, la base mise en œuvre pour la formation *in situ* des ions orthovanadate à partir du sel de métavanadate est une source de cations tétraalkylammonium. Il peut s'agir par exemple d'un hydroxyde de tétraalkylammonium, par exemple de l'hydroxyde de tétraméthylammonium.

La mise en œuvre d'un sel de métavanadate tel que par exemple le métavanadate d'ammonium pour la synthèse des nanoparticules selon l'invention s'avère particulièrement avantageuse, comparativement à la mise en œuvre de l'orthovanadate de sodium, en ce qu'elle permet de s'affranchir des problèmes de reproductibilité liés aux variations de la teneur en carbonate de l'orthovanadate de sodium.

A titre d'exemple, la réaction de synthèse à partir du métavanadate d'ammonium et de l'hydroxyde de tétraméthylammonium est représentée en exemple 1.

Il peut être également envisagé d'utiliser du métavanadate d'alkylammonium (non disponible commercialement) et une autre base forte, comme l'hydroxyde d'ammonium.

De manière avantageuse, la présence d'ions de type tétraalkylammonium en surface des nanoparticules permet non seulement une grande stabilité colloïdale à l'issue de la synthèse mais également une stabilité des particules formées tout au long de la synthèse et ainsi une bien meilleure reproductibilité du procédé de synthèse lui-même.

Le procédé de préparation des particules selon l'invention peut plus particulièrement mettre en œuvre au moins les étapes suivantes, consistant en :

(i) disposer d'une solution aqueuse, notée solution (1), comprenant des ions orthovanadate ($VO_4^{3-}$), et éventuellement des ions phosphates ($PO_4^{3-}$), et des cations tétraalkylammonium ;

(ii) ajouter à la solution aqueuse (1), une solution aqueuse, dite solution (2), comprenant lesdits précurseurs des éléments A et Ln, en particulier sous forme de sels, notamment de nitrates, dans des conditions propices à la formation par co-précipitation des nanoparticules de formule (I) ; et

(iii) récupérer lesdites nanoparticules de formule (I) à la surface desquelles sont localisés des cations tétraalkylammonium, formées à l'issue de l'étape (ii).

La solution aqueuse (1) peut être plus particulièrement préparée par mélange d'au moins un sel de métavanadate, en particulier du métavanate d'ammonium et d'au moins une base, source de cations tétraalkylammonium, par exemple d'un hydroxyde de tétraalkylammonium.

Dans le cas des ions phosphates, on ajoute un sel de phosphate, tel que le phosphate de sodium ou le phosphate d'ammonium.

Ainsi, selon une variante de réalisation particulièrement avantageuse, le procédé de l'invention comprend au moins les étapes consistant en :

(i) préparer une solution aqueuse (1) par mélange, en milieu aqueux, d'un sel de métavanadate, en particulier du métavanadate d'ammonium ($NH_4VO_3$), et éventuellement un sel de phosphate, et d'une base, source de cations tétraalkylammonium, en particulier un hydroxyde de tétraalkylammonium ;

(ii) ajouter à la solution aqueuse (1), une solution aqueuse (2) comprenant lesdits précurseurs des éléments A et Ln, en particulier sous forme de sels, notamment de nitrates ; dans des conditions propices à la formation par co-précipitation desdites nanoparticules de formule (I) ; et

(iii) récupérer les nanoparticules de formule (I) à la surface desquelles sont localisés des cations tétraalkylammonium, formées à l'issue de l'étape (ii).

Selon un mode de réalisation particulier, l'ajout en étape (ii) de la solution (2) à la solution (1) est opéré goutte à goutte.

Selon une autre variante de réalisation, la solution (2) peut être ajoutée à la solution (1) en une seule fois, et non pas au goutte à goutte.

Le milieu aqueux des solutions (1) et (2) est plus particulièrement formé d'eau.

Dans un mode de réalisation particulier, la solution aqueuse (2) contenant les précurseurs des éléments A et Ln peut également comprendre des complexants de ces éléments, tel que le citrate, par exemple le citrate de tétraalkylammonium.

Il appartient à l'homme du métier d'ajuster de manière adéquate les quantités des différents réactifs, en particulier des précurseurs des ions orthovanadate, éventuellement phosphate, et desdits éléments A et Ln, au regard de la nature souhaitée pour la nanoparticule de formule (I) selon l'invention.

En particulier, les proportions stœchiométriques des différents réactifs selon les formules (I), (I'), (II), (II'), (III) et (III') sont à respecter. Lorsque l'on prévoit une excitation optique des ions Ln par excitation directe, i.e. en résonance avec les états électroniques de ces ions, et non par excitation de la matrice $AVO_{4(1-y)}(PO_4)_y$ et transfert d'énergie ultérieur vers ces ions, comme évoqué précédemment, il est préférable de disposer d'une valeur élevée de x, de préférence entre 0,2 et 0,6, et de préférence 0,4.

De manière avantageuse, le procédé de préparation des particules de l'invention ne requiert aucun chauffage de la solution, à la différence notamment des méthodes hydrothermales proposées dans les publications [13]-[18]. En particulier, l'ensemble des étapes (i) à (iii) pour la synthèse des particules selon l'invention peut être avantageusement opéré à température ambiante (20-25°C).

Néanmoins, des procédés de préparation à plus haute température, par exemple de type hydrothermal, peuvent produire des nanoparticules de cristallinité similaire, à condition que la température n'excède pas 600°C, ce qui permet ainsi d'accéder à des nanoparticules présentant une cristallinité imparfaite comme évoqué précédemment, et ainsi obtenir un rendement quantique élevé.

L'étape (iii) consiste plus particulièrement à purifier la solution de particules obtenues, notamment pour éliminer l'excès de contre-ions.

Les étapes de purification peuvent plus particulièrement comprendre des étapes de dialyse ou de centrifugation et redispersion des particules en milieu aqueux, par exemple par sonification.

Les particules peuvent être redispersées dans un milieu aqueux, en particulier dans l'eau. Comme évoqué précédemment, la suspension colloïdale aqueuse des particules de l'invention présente une très bonne stabilité, même après stockage pendant plusieurs mois.

Selon un autre de ses aspects, l'invention concerne encore les particules obtenues à l'issue du procédé tel que décrit précédemment.

Selon une variante de réalisation particulière, l'invention concerne un procédé de préparation de particules formées en tout ou partie d'une nanoparticule de formule $Y_{1-x}Eu_xVO_4$ (III), avec $0<x<1$, comprenant au moins les étapes consistant en :

(i) disposer d'une solution aqueuse (1), comprenant des ions orthovanadate et des cations tétraalkylammonium, ladite solution aqueuse (1) étant de préférence obtenue à partir du mélange, en milieu aqueux, du métavanadate d'ammonium ($NH_4VO_3$) et d'un hydroxyde de tétraalkylammonium ;
(ii) ajouter à la solution aqueuse (1), une solution aqueuse, dite solution (2), comprenant des précurseurs de Y et Eu, en particulier des nitrates d'yttrium et d'europium, dans des conditions propices à la formation par co-précipitation des nanoparticules de formule (III) ; et
(iii) récupérer lesdites nanoparticules de formule (III) à la surface desquelles sont localisés des cations tétraalkylammonium, formées à l'issue de l'étape (ii).

Selon un mode de réalisation particulier, le procédé peut comprendre en outre une ou plusieurs étapes de couplage (greffage) des particules obtenues avec un ou plusieurs agents de ciblage, en vue notamment de leur utilisation pour une méthode de diagnostic *in vitro, ex vivo,* ou *in vivo.*

Il appartient à l'homme du métier de mettre en œuvre les méthodes de couplage/greffage adaptées pour préparer de manière adéquate les particules en vue de leur mise en œuvre comme sonde de diagnostic. La quantité d'agent(s) de ciblage mise en œuvre est ajustée au regard de la quantité de particules.

L'agent de ciblage peut être greffé directement, ou *via* un espaceur (encore désigné sous les termes « linker » ou « spaceur »), à la nanoparticule.

Les méthodes de couplage (encore appelé greffage) des particules à des biomolécules sont bien connues de l'homme de l'art. Il s'agit en général d'un couplage par liaison covalente, par complexation de surface, par interactions électrostatiques, par encapsulation, ou par adsorption. Dans certains cas, dont le cas d'un couplage par liaison covalente, les particules peuvent être préalablement fonctionnalisées par un ou plusieurs groupements chimiques capables ensuite de réagir avec un autre groupement chimique porté par l'agent de ciblage pour former une liaison covalente.

Comme exemple de groupements chimiques pouvant être présents à la surface des nanoparticules, on peut citer les groupements carboxyle, amino, thiol, aldéhyde et époxy.

Par exemple, les nanoparticules de l'invention peuvent être fonctionnalisées en surface avec du citrate, comme illustré dans l'exemple 4 qui suit.

Selon un autre mode de réalisation particulier, les nanoparticules de l'invention peuvent être fonctionnalisées en surface par du polyacide acrylique (PAA). Dans le cas d'une fonctionnalisation avec du PAA, le nombre de liaisons de coordination (liaisons datives) formé par chaque molécule de PAA est d'autant plus important que le PAA est long. Le PAA peut par exemple présenter un degré de polymérisation allant de 3 à 10000.

De manière avantageuse, la fonctionnalisation des nanoparticules avec du PAA conduit à des nanoparticules fonctionnalisées, et à des nanoparticules résultant du couplage de ces nanoparticules fonctionnalisées avec un ou plusieurs agents de ciblage, qui présentent d'excellentes propriétés en termes de stabilité dans le temps.

Des groupements amino peuvent être apportés par des molécules comme les organosilanes aminés, tel que l'aminotriéthoxysilane (APTES). L'avantage de l'APTES réside dans le fait qu'il forme *via* des liaisons covalentes une capsule autour de la nanoparticule. Les amines apportées par l'APTES sont ainsi très stables dans le temps. Les groupements amino peuvent être transformés en groupements carboxyle par réaction avec de l'anhydride succinique.

Des groupements carboxyle peuvent être apportés par des molécules telles que l'acide citrique ou un polyacide acrylique (PAA).

Les groupements carboxyle peuvent être activés selon toute technique connue de l'homme de l'art, en particulier par réaction avec le 1-éthyl-3-(3-diméthylaminopropyl)carbodiimide (EDC) et le N-hydroxysuccinimide (NHS), pour réagir ensuite avec les fonctions amines à la surface d'un polypeptide et former une liaison amide covalente, lorsque l'agent de ciblage est une protéine ou un anticorps.

La fonctionnalisation des nanoparticules par de l'APTES peut se faire avantageusement suite au recouvrement des nanoparticules par une couche de silice.

## APPLICATIONS

Comme précisé précédemment, les particules synthétisées selon l'invention, en particulier couplées à au moins un agent de ciblage, trouvent une application particulièrement avantageuse en tant que sondes ou bio-marqueurs dans des échantillons biologiques.

La présente invention concerne, selon un autre de ses aspects, l'utilisation des particules décrites précédemment ou obtenues selon le procédé décrit précédemment, en particulier couplées à au moins un agent de ciblage, ou d'une suspension aqueuse colloïdale de ces particules, à titre d'agent de diagnostic, c'est-à-dire pour la détection et/ou quantification d'une substance d'intérêt biologique ou chimique.

Les nanoparticules peuvent être notamment utilisées à titre d'agent de diagnostic *in vitro* ou *ex vivo,* autrement dit pour la détection et/ou quantification d'une substance d'intérêt biologique ou chimique au sein d'un échantillon, en particulier un échantillon biologique.

Les particules de l'invention peuvent être couplées avec des agents de ciblage variés.

Par « agent de ciblage », on entend un composé permettant une liaison avec une substance d'intérêt biologique ou chimique, et dont l'identification est recherchée.

Les sondes selon l'invention sont parfaitement adaptées à une grande diversité de ciblages biologiques, les spécificités étant dépendantes de la nature du ou des agents de ciblage greffés à la surface de la nanoparticule.

L'agent de ciblage peut être plus particulièrement un anticorps polyclonal ou monoclonal, un fragment d'anticorps, un oligonucléide, un peptide, une hormone, un ligand, une cytokine, un peptidomimétique, une protéine, un carbohydrate, une protéine modifiée chimiquement, un acide nucléique modifié chimiquement, un carbohydrate modifié chimiquement qui cible une protéine de surface cellulaire connue, un aptamère, un assemblage de protéines et d'ADN/ARN ou un chloroalcane utilisé par les marquages de type HaloTag.

Selon un mode de réalisation particulier, il s'agit d'un anticorps ou fragment d'anticorps.

Des fragments d'anticorps appropriés comprennent au moins un domaine variable d'une immunoglobuline, tels que des domaines variables simples Fv, scFv, Fab, (Fab')$^2$ et autres fragments protéolytiques ou des « nanobody » (anticorps à domaine unique tels que les fragments $V_HH$ obtenus à partir d'anticorps de camélidés ou $V_{NAR}$ obtenus à partir d'anticorps de poissons cartilagineux).

Le terme « anticorps » selon l'invention inclut des anticorps chimériques ; des anticorps humains ou humanisés, des anticorps recombinants et modifiés, des anticorps conjugués, et leurs fragments.

Selon un mode de réalisation particulier, les anticorps ou fragments d'anticorps mis en œuvre selon l'invention ciblent des marqueurs spécifiques de cellules cancéreuses.

L'agent de ciblage peut également être dérivé d'une molécule connue pour lier un récepteur de surface cellulaire. Par exemple, le fragment de ciblage peut dériver de lipoprotéines de faible densité, transferrine, EGF, insuline, PDGF, enzymes fibrinolytiques, anti-HER2, anti-HER3, anti-HER4, annexines, interleukines, interférons, érythropoïétines ou facteurs stimulant les colonies.

Les particules couplées à un agent de ciblage selon l'invention peuvent être utilisées dans les méthodes de diagnostic *in vitro, ex vivo,* ou *in vivo,* impliquant une reconnaissance d'un couple ligand/anti-ligand, par exemple biotine ou composés biotinylés/avidine ou streptavidine, haptène/anticorps, antigène/anticorps, peptide/anticorps, sucre/lectine, polynucléotide/complémentaire de polynucléotide, etc. étant entendu que l'un des éléments de ces couples constitue la molécule d'intérêt biologique, ou peut également être couplé à la molécule d'intérêt biologique.

Un exemple de couplage de particules selon l'invention de type $Y_{0,6}Eu_{0,4}VO_4$ à une protéine, la streptavidine, et à un anticorps est donné en exemple 4 qui suit.

Les particules selon l'invention permettent de déterminer, par exemple dans un échantillon biologique, la présence de la molécule d'intérêt qui s'associera à l'agent de ciblage, la révélation de l'association étant réalisée grâce aux propriétés de luminescence des particules de l'invention, après notamment l'élimination des agents de ciblage qui ne se seront pas liés à la molécule d'intérêt.

Les particules de l'invention peuvent être ainsi mises en œuvre pour la détection et/ou quantification de concentrations en biomarqueurs, anticorps, ADN, ARN, etc. dans des échantillons biologiques.

Les échantillons biologiques peuvent par exemple être choisi dans la liste comprenant le sang, le sérum, le plasma, la salive, l'urine, le liquide cérébro-spinal, etc..

Les méthodes de diagnostic *in vitro* ou *ex vivo,* pouvant mettre en œuvre les particules de l'invention sont largement connues de l'homme du métier. A titre d'exemples, elles peuvent comprendre les techniques suivantes : immuno-essai, immunomarquage, immuno-histochimie, immuno-cytochimie, immunomarquage, technique du Western-blot, technique dite du « dot blot », cytométrie en flux, méthode dite « FACS » pour « fluorescence-activated cell sorting », et plus généralement tout type de détection de biomolécules sur la surface de cellules vivantes ou fixées ou sur tranches de tissus cryogéniques ou non, ELISA, ELOSA, ELISPOT, analyse multiplexe/multidétection, hybridation *in situ* en fluorescence ou « FISH » (pour « Fluorescence *in situ* hybridization »), etc...

Les particules de l'invention peuvent encore être mises en œuvre dans une méthode de détection d'ADN par hybridation ou de détection d'ARN par RT-PCR suivie d'hybridation.

Les supports pour l'analyse *in vitro* ou *ex vivo* peuvent être de diverses natures. Il peut s'agir de lamelles, multi-puits, microplaques, gels, membranes, bandelettes, microcanaux, etc..

La détection peut être opérée par tout dispositif usuel, comprenant d'une manière générale, une source de rayonnement pour l'excitation des particules, par exemple un dispositif d'illumination laser, en particulier monochromatique, et un moyen de détection de l'intensité lumineuse émise par les particules, par exemple sous la forme d'une caméra telle qu'une caméra CCD, CCD à multiplication d'électrons (EM-CCD), ou photo-détecteur unique notamment du type photomultiplicateur ou photodiode.

Les maladies pouvant être diagnostiquées avec les particules de l'invention ne sont pas limitées et comprennent toutes maladies révélées par la présence d'un ou plusieurs marqueurs spécifiques de la maladie, du type molécule d'intérêt biologique, pour lequel il existe un partenaire de liaison spécifique.

A titre d'exemples, on peut citer les maladies infectieuses (bactériennes, parasitaires, ou virales, telles que le SIDA), les maladies inflammatoires et auto-immunes, les maladies cardiologiques, neurologiques, ou oncologiques (par exemple, les cancers solides tels que le cancer du sein ou de la prostate).

L'invention concerne encore, selon un autre de ses aspects, un kit de diagnostic, *in vitro, ex vivo* ou *in vivo*, comprenant au moins des particules selon l'invention ou une suspension colloïdale aqueuse de ces particules.

En particulier, elle concerne un kit de détection et/ou de quantification *in vitro* ou *ex vivo* d'une substance d'intérêt biologique ou chimique dans un échantillon, comprenant au moins des particules selon l'invention ou une suspension aqueuse colloïdale de ces particules.

Le kit de détection peut plus particulièrement comprendre :

- un support à la surface duquel est immobilisé un agent de ciblage de l'analyte à détecter/quantifier, par exemple un premier anticorps, dit « anticorps de capture », spécifique à la substance d'intérêt biologique ou chimique à détecter/quantifier ; et
- un ou plusieurs récipients comprenant au moins des nanoparticules selon l'invention et au moins un agent de ciblage sous forme couplée ou non aux nanoparticules.

Le support peut être de tout type, notamment en verre ou en plastique, tel qu'une plaque, une colonne, un gel, des billes magnétiques, etc., comme évoqué précédemment. Il s'agit avantageusement d'une plaque multi-puits. L'immobilisation est typiquement réalisée par adsorption ou par liaison covalente sur la surface des puits.

Selon une première alternative, le kit peut comprendre des nanoparticules selon l'invention déjà couplées à un agent de ciblage, en particulier à des anticorps, appelés « anticorps de révélation » pour les distinguer des anticorps de capture immobilisés au niveau du support.

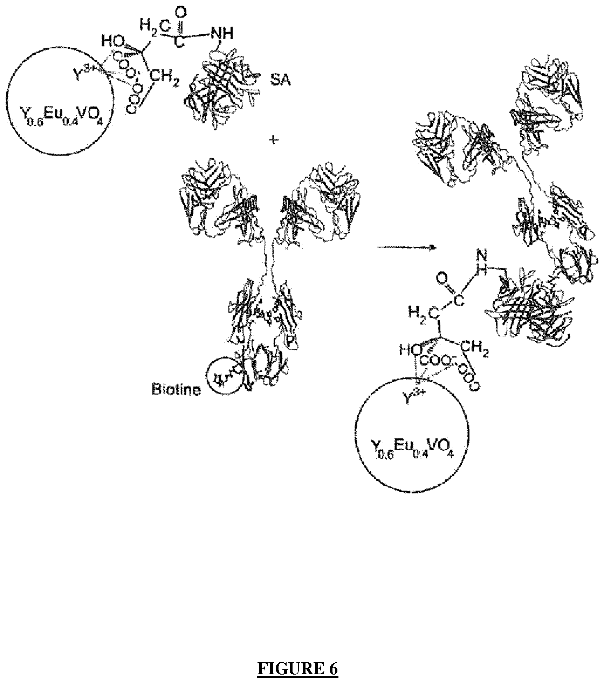

Les nanoparticules couplées aux anticorps, mises en œuvre dans un kit selon l'invention, peuvent être par exemple obtenues, comme décrit en exemple 4 et schématisé en figure 6, par couplage des nanoparticules couplées avec la streptavidine, avec des anticorps biotinylés, ou encore directement par couplage des anticorps sur les nanoparticules fonctionnalisées avec du citrate ou du polyacide acrylique.

Alternativement, le kit peut comprendre plusieurs récipients comprenant, de manière isolée, les nanoparticules selon l'invention et l'agent de ciblage, et à partir desquels le praticien peut préparer des nanoparticules selon l'invention couplées à l'agent de ciblage.

Par exemple, le kit peut comprendre un premier récipient comprenant les nanoparticules de l'invention fonctionnalisées avec du citrate ou du polyacide acrylique, et un deuxième récipient comprenant les anticorps biotinylés, la préparation des nanoparticules couplées aux anticorps impliquant l'activation des fonctions carboxyliques contenues dans le premier récipient comme décrit en exemple 4 suivie du mélange du contenu des premier et deuxième récipients.

Bien entendu, un kit de détection selon l'invention comprenant des particules selon l'invention n'est pas limité aux variantes de kits décrites ci-dessus.

Les particules de l'invention peuvent encore être mises en œuvre, selon un autre mode de réalisation, comme agent de diagnostic destiné à l'imagerie optique *in vivo* ou encore comme agent amplificateur de traitements *in vivo* par irradiation.

Le présent texte décrit ainsi des particules luminescentes selon l'invention, couplées ou non à au moins un agent de ciblage, pour leur utilisation *in vivo* en tant qu'agent de diagnostic destiné à l'imagerie optique ou à l'imagerie par résonance magnétique lorsque les particules comportent du gadolinium, ou à l'imagerie par rayons X. En particulier, elles peuvent être utilisées comme agent de contraste IRM.

Les nanoparticules de l'invention peuvent encore être utilisées en radiothérapie du fait de leur pouvoir sensibilisant.

Le présent texte décrit ainsi des particules luminescentes selon l'invention, couplées ou non à au moins un agent de ciblage pour leur utilisation *in vivo* en tant qu'agent amplificateur *in vivo* dans le traitement de cancers à l'aide d'une irradiation ionisante (électrons, protons, photons X ou $\gamma$,...).

Les nanoparticules sont ainsi utilisées pour renforcer la puissance destructrice de la radiothérapie dans les cellules tumorales, tout en diminuant la toxicité pour les tissus sains.

Les exemples et figures présentés ci-dessous sont uniquement donnés à titre illustratif et non limitatif de l'invention.

**Figures**

Figure 1 : Images obtenues par microscopie électronique à transmission (MET) des nanoparticules obtenues selon l'exemple 1 (Barre d'échelle : 60 nm (figure la) et 5 nm (figure 1b), respectivement) ;

Figure 2 : Histogramme de taille des nanoparticules déterminé à partir d'images de MET pour un ensemble d'environ 300 nanoparticules selon l'exemple 1 ;

Figure 3 : Diffractogrammes des rayons X obtenus pour les nanoparticules synthétisées selon l'exemple 1 (trait noir), et 3 (trait gris) ;

Figure 4 : Représentation schématique des nanoparticules de l'invention fonctionnalisées avec du citrate (figure 4a) ou avec du polyacide acrylique (PAA) (figure 4b). A des fins de clarté, une seule molécule de citrate ou de PAA est représentée. Il est entendu que chaque nanoparticule peut comporter sur sa surface de nombreuses molécules de citrate ou de PAA.

Figure 5 : Représentation schématique des réactions pour le couplage d'une nanoparticule selon l'invention avec la streptavidine, selon l'exemple 4 ;

Figure 6 : représentation schématique du couplage des nanoparticules couplées avec la streptavidine, avec un anticorps biotinylé.

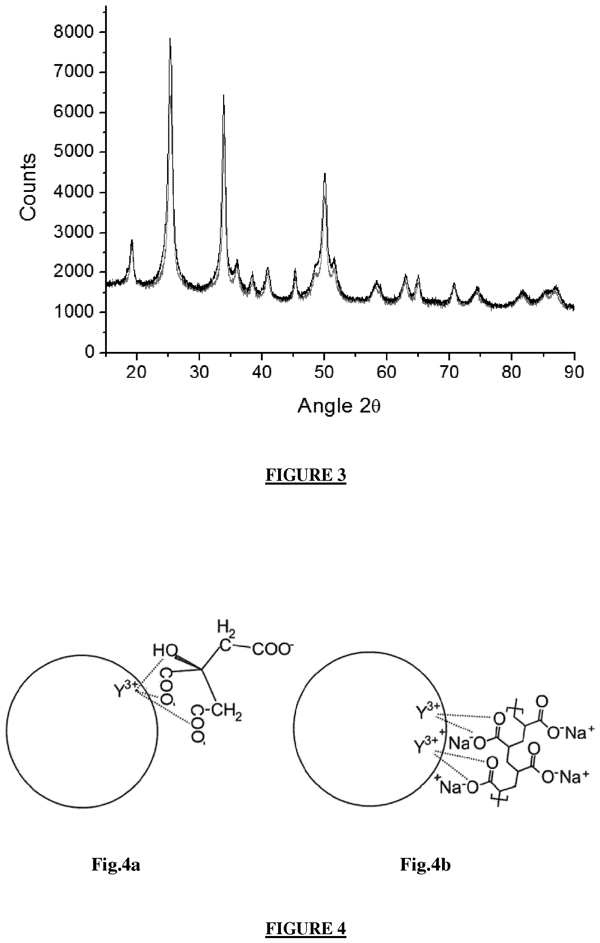

Il est entendu que les figures 4 à 6 sont des schémas de principe, notamment en ce qui concerne la structure des molécules de surface qui peuvent présenter plusieurs configurations autres que celle montrée sur ces figures.

## EXEMPLES

## EXEMPLE 1

### Synthèse de nanoparticules $Y_{0,6}Eu_{0,4}VO_4$ selon l'invention

Comme source d'ions de métavanadate $VO_3^-$, on utilise du métavanadate d'ammonium $NH_4VO_3$, l'orthovanadate $VO_4^{3-}$ étant obtenu *in situ* suite à une réaction avec une base, ici l'hydroxyde de tétraméthylammonium, $N(CH_3)_4OH$. Des nitrates d'yttrium et d'europium ont été utilisés comme sources d'ions $Y^{3+}$ et $Eu^{3+}$.

Une solution aqueuse de 10 mL de $NH_4VO_3$ à 0,1 M et 0,2 M de $N(CH_3)_4OH$ (solution 1) est fraichement préparée.

Un volume de 10 mL d'une autre solution de $Y(NO_3)_3$ et de $Eu(NO_3)_3$ à 0,1 M en ions ($Y^{3+} + Eu^{3+}$) est ajoutée goutte à goutte à l'aide d'un pousse seringue dans la solution 1 à un flux de 1 mL/min.

Le rapport en concentration molaire entre $Y(NO_3)_3$ et $Eu(NO_3)_3$ est choisi en fonction du rapport entre ions $Y^{3+}$ et $Eu^{3+}$ souhaité dans la nanoparticule, typiquement le rapport molaire $Y^{3+}$:$Eu^{3+}$ est de 0,6 : 0,4.

Dès l'ajout de la solution $Y(NO_3)_2/Eu(NO_3)_3$, la solution devient diffusante et apparaît blanche/laiteuse sans formation de précipité. La synthèse se poursuit jusqu'à l'ajout total de la solution $Y(NO_3)_2/Eu(NO_3)_3$.

La solution finale de 20 mL doit à présent être purifiée pour éliminer l'excès de contre-ions. Pour ce faire, des centrifugations (typiquement trois) à 11000 g (Sigma 3K10, Bioblock Scientific) pendant 80 minutes suivies chacune d'une redispersion par sonification (Branson Sonifier 450 fonctionnant à 50 % à une puissance de 400 W) sont utilisées jusqu'à atteindre une conductivité strictement inférieure à 100 $\mu S.cm^{-1}$.

La conductivité est mesurée à l'aide d'un conductimètre de chimie.

La synthèse des nanoparticules $Y_{0,6}Eu_{0,4}VO_4$ à la surface desquelles sont immobilisés les cations tétraméthylammonium peut être schématisée comme suit :

$$NH_4VO_3 + 2\,(N(CH_3)_4)OH \leftrightarrows VO_4^{3-} + 2\,N(CH_3)_4^+ + NH_4^+$$

$$VO_4^{3-} + 2\,N(CH_3)_4^+ + NH_4^+ + 0.6\,Y(NO_3)_3 + 0.4\,Eu(NO_3)_3 \rightarrow Y_{0.6}Eu_{0.4}VO_4 + 2\,N(CH_3)_4^+ + NH_4^+ + 3NO_3^-$$

Deux essais de synthèse (« synthèse 1 » et « synthèse 2 ») sont effectués.

Résultat

L'observation visuelle de la solution de nanoparticules selon l'invention, après avoir été laissée au repos pendant 16 heures dans un flacon, montre une solution uniformément diffusante.

La solution finale reste très stable dans l'eau, même après plusieurs mois dans le pH final de la synthèse (environ pH 7-9). La solution reste stable y compris dans le milieu de synthèse (avant l'élimination de l'excès de contre-ions), de force ionique pourtant élevée (>0,1 M).

Le potentiel zêta des nanoparticules est déterminé avec un appareil DLS-Zeta Potential (Zetasizer Nano ZS90, Malvern). Les résultats des potentiels zêta mesurés pour les nanoparticules des deux synthèses sont rassemblés dans le tableau 1 ci-après.

Pour les observations par microscopie électronique à transmission (MET), des solutions diluées de nanoparticules sont déposées sur une grille en carbone. Les observations sont réalisées à l'aide d'un microscope Philips CM30 fonctionnant à 300 kV avec une résolution de 0,235 nm.

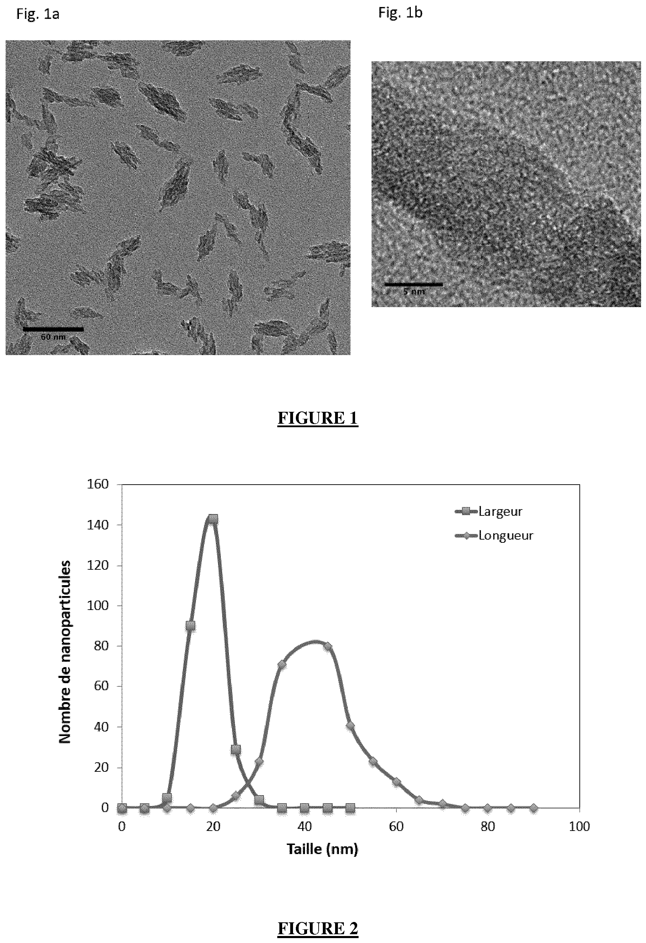

L'observation des nanoparticules par MET (figure 1) montre que les nanoparticules sont de forme éllipsoïde allongée. Les dimensions des nanoparticules sont déterminées à partir d'images de MET pour un ensemble d'environ 300 nanoparticules (figure 2). Les nanoparticules de l'invention présentent une longueur du grand axe, notée $a$, comprise entre 20 et 60 nm, avec une valeur moyenne d'environ 40 nm, et une longueur du petit axe, notée b, comprise entre 10 et 30 nm, avec une valeur moyenne d'environ 20 nm.

Les images de MET (figure 1) ne permettent pas de distinguer des plans cristallins, ce qui est probablement attribuable au fait que la nanoparticule est constituée de plusieurs cristallites de taille plus faible que la taille de la nanoparticule. La nature majoritairement cristalline et polycristalline des nanoparticules est confirmée par des expériences de diffraction de rayons X.

Le diffractogramme des rayons X obtenu à l'aide d'un diffractomètre Philips X-pert avec la raie $K_{\alpha 1}$ du Cuivre ($\lambda$=1,5418 Å), est représenté en figure 3. L'intensité diffractée est enregistrée en utilisant un détecteur X'Celerator area detector (PANalytical).

La longueur de cohérence dans une direction cristallographique et donc la taille moyenne des cristallites constituant la nanoparticule dans cette direction cristallographique peut être estimée à partir de la largeur des pics dans le diffractogramme RX par application de la formule de Scherrer. Les valeurs de longueur de cohérence obtenues pour les différentes directions cristallographiques sont comprises entre 3 et 40 nm. La longueur de cohérence dans au moins une direction cristallographique étant inférieure à la dimension de la nanoparticule dans cette direction, on peut en déduire que les nanoparticules sont d'une cristallinité imparfaite (polycristallinité, défauts ou porosité). Dans la direction (200) (Fig. 3, pic à $2\theta \cong 25°$), la longueur de cohérence est de 10,2 nm, légèrement plus faible que la longueur de cohérence pour les nanoparticules de l'exemple 3 (11,1 nm).

### EXEMPLE 2 (hors invention)

### Synthèse de nanoparticules $Y_{0,6}Eu_{0,4}VO_4$ à partir de métavanadate de sodium, sans contre-ions volumineux

Pour la synthèse par co-précipitation de sels métalliques, des nitrates d'yttrium et d'europium ont été utilisés comme sources d'ions $Y^{3+}$ et $Eu^{3+}$. Comme source d'ions de métavanadate $VO_3^-$, on utilise du métavanadate de sodium $NaVO_3$, l'orthovanadate $VO_4^{3-}$, étant obtenu *in situ* suite à une réaction avec une base, ici l'hydroxyde de sodium, NaOH.

Une solution aqueuse de 10 mL de $NaVO_3$ à 0,1M et de 0,2M de NaOH (solution 1) est fraichement préparée.

Un volume de 10 mL d'une autre solution de $Y(NO_3)_3$ et de $Eu(NO_3)_3$ à 0,1 M en ions ($Y^{3+}$ + $Eu^{3+}$) est ajoutée goutte à goutte à l'aide d'un pousse seringue dans la solution 1 à un débit de 1 mL/min.

Le rapport en concentration molaire entre $Y(NO_3)_3$ et $Eu(NO_3)_3$ est choisi en fonction du rapport entre ions $Y^{3+}$ et $Eu^{3+}$ souhaité dans la nanoparticule, typiquement le rapport molaire $Y^{3+}$ :$Eu^{3+}$ est de 0,6 : 0,4.

Dès l'ajout de la solution $Y(NO_3)_2$/$Eu(NO_3)_3$, un précipité laiteux apparaît. La synthèse se poursuit jusqu'à l'ajout total de la solution $Y(NO_3)_2$/$Eu(NO_3)_3$.

La solution finale de 20 mL doit à présent être purifiée pour éliminer l'excès de contre ions. Pour ce faire, des centrifugations (typiquement trois) à 11000 g (Sigma 3K10, Bioblock Scientific) pendant 80 minutes suivies chacune d'une redispersion par sonification (Branson Sonifier 450 fonctionnant à 50 % avec une puissance de 400 W) sont utilisées jusqu'à atteindre une conductivité strictement inférieure à 100 $\mu$S.cm$^{-1}$.

### Résultat

Une comparaison de la solution obtenue avec la solution de nanoparticules selon l'invention obtenue en exemple 1 (pour une même concentration finale en ions vanadate), après 16 heures au repos, montre qu'à la différence de la solution de l'exemple 1, la solution est moins diffusante (moins blanche) ; un léger dépôt s'est formé au fond du flacon.

Le potentiel zêta des nanoparticules, déterminé comme décrit en exemple 1, est présenté dans le tableau 1 ci-après.

### EXEMPLE 3 (hors invention)

### Synthèse de nanoparticules $Y_{0,6}Eu_{0,4}VO_4$ à partir d'orthovanadate de sodium, sans contre-ions volumineux

Pour la synthèse par co-précipitation de sels métalliques, des nitrates d'yttrium et d'europium ont été utilisés comme sources d'ions $Y^{3+}$ et $Eu^{3+}$. Comme source d'ions d'orthovanadate, $VO_4^{3-}$, est mis en œuvre de l'orthovanadate de sodium $Na_3VO_4$.

Une solution aqueuse de 10 mL de $Na_3VO_4$ à 0,1M (solution 1) est fraichement préparée. Le pH est mesuré et ajusté si nécessaire pour atteindre une valeur comprise entre 12,6 et 13.

Un volume de 10 mL d'une autre solution de $Y(NO_3)_3$ et de $Eu(NO_3)_3$ à 0,1 M en ions ($Y^{3+}$ + $Eu^{3+}$) est ajoutée goutte à goutte à l'aide d'un pousse seringue dans la solution 1 à un flux de 1 mL/min.

Le rapport en concentration molaire entre $Y(NO_3)_3$ et $Eu(NO_3)_3$ est choisi en fonction du rapport entre ions $Y^{3+}$ et $Eu^{3+}$ souhaité dans la nanoparticule, typiquement le rapport molaire $Y^{3+}$ :$Eu^{3+}$ est de 0,6 : 0,4.

Dès l'ajout de la solution $Y(NO_3)_2$/$Eu(NO_3)_3$, un précipité laiteux apparaît. La synthèse se poursuit jusqu'à l'ajout total de la solution $Y(NO_3)_2$/$Eu(NO_3)_3$.

La solution finale de 20 mL doit à présent être purifiée pour éliminer l'excès de contre-ions. Pour ce faire, des centrifugations (typiquement trois) à 11000 g (Sigma 3K10, Bioblock Scientific) pendant 80 minutes suivies chacune d'une redispersion par sonification (Branson Sonifier 450 fonctionnant à 50 % à une puissance de 400 W) sont utilisées jusqu'à atteindre une conductivité strictement inférieure à 100 $\mu$S.cm$^{-1}$.

Résultats

Une comparaison de la solution obtenue avec la solution de nanoparticules selon l'invention obtenue en exemple 1 (pour une même concentration finale en ions vanadate), après 16 heures au repos, montre qu'à la différence de la solution de l'exemple 1, la majorité des nanoparticules se sont déposées au fond du flacon ; la partie haute de la solution est presque transparente ce qui indique une absence de diffusion et donc de nanoparticules en solution.

Le potentiel zêta des nanoparticules, déterminé comme décrit en exemple 1, est présenté dans le tableau 1 ci-après.

Le diffractogramme des rayons X obtenu à l'aide d'un diffractomètre Philips X-pert est représenté en figure 3.

De même que pour l'exemple 1, la taille moyenne des cristallites constituant la nanoparticule dans la direction cristallographique (200) peut être estimée par application de la formule de Scherrer, et donne une valeur comprise entre 3 et 40 nm. La taille des cristallites étant globalement inférieure aux dimensions de la nanoparticule, on peut en déduire que les nanoparticules synthétisées selon l'exemple 3 sont également polycristallines.

| Exemples | Exemple 1 (conforme à l'invention) | | Exemple 2 (hors invention) | Exemple 3 (hors invention) |
|---|---|---|---|---|
| | Synthèse 1 | Synthèse 2 | | |
| Conductivité ($\mu$S/cm) | 93 | 80 | 53 | 66 |
| pH | 4,8 | 5,0 | 5,4 | 9,6 |
| Potentiel zêta $\zeta$* | -33,3 | -34,6 | -2,30 | -18,00 |
| * mesures du potentiel zêta effectuées après purification pour avoir une conductivité < 100 $\mu$S.cm$^{-1}$ et après dilution dans de l'eau Milli-Q® acide. | | | | |

**TABLEAU 1** : comparaison du potentiel zêta $\zeta$ des nanoparticules obtenues selon l'exemple 1 conforme à l'invention (synthèse à base de métavanadate d'ammonium avec contre-ions volumineux), et selon les exemples 2 et 3 non conformes à l'invention (synthèse à base de métavanadate de sodium sans contre-ions volumineux et synthèse à base d'orthovanadate sans contre-ions volumineux).

La comparaison des potentiels zêta des nanoparticules obtenues selon les exemples 1 à 3 confirment que les nanoparticules selon l'invention (exemple 1) présentent une stabilité colloïdale améliorée avec |$\zeta$|>30 mV comparativement aux nanoparticules obtenues sans cations tétraalkylammonium (exemples 2 et 3).

Une synthèse alternative des nanoparticules $Y_{0,6}Eu_{0,4}VO_4$ selon l'invention a été effectuée suivant le même protocole que la synthèse de l'exemple 1, avec pour seule différence, le fait que la solution de 10 mL de $Y(NO_3)_3$ et de $Eu(NO_3)_3$ à 0,1 M en ions ($Y^{3+}$ + $Eu^{3+}$) n'est pas ajoutée à la solution 1 goutte à goutte, mais en une seule fois.

A l'issue de la synthèse, après purification, le potentiel zêta est de -35 mV à un pH de 7,8.

## EXEMPLE 4

**Couplage des nanoparticules avec la protéine streptavidine**

i. Greffage du citrate à la surface des nanoparticules

A l'issue de la synthèse des nanoparticules selon l'exemple 1, on centrifuge la solution de nanoparticules à 17 000 g pendant 3 minutes, pour précipiter les éventuels agrégats de nanoparticules et on récupère le surnageant.

On prélève environ 250 µL de particules $Y_{0,6}Eu_{0,4}VO_4$ de concentration 5 mM en ions vanadate, et on les disperse dans 1 mL d'une solution d'eau distillée contenant l'ion citrate (concentration 0,2 M).

La solution est ensuite sonifiée pendant 5 minutes puis centrifugée à 17 000 g pendant 3 minutes. Cette étape est répétée 3 fois.

A l'issue de ce greffage, les particules sont dispersées dans de l'eau distillée, solvant dans lequel elles sont stables.

La fonctionnalisation des nanoparticules par du citrate peut être remplacée par une fonctionnalisation par du PAA (par exemple de degré de polymérisation compris entre 3 et 10000), en mettant en œuvre un sel de PAA, comme par exemple un sel de sodium ou d'ammonium.

En figure 4, sont représentés de manière schématique les nanoparticules de l'invention fonctionnalisées avec (a) du citrate et (b) du polyacide acrylique.

ii. Couplage des nanoparticules avec la streptavidine

On centrifuge les nanoparticules (NPs) greffées avec les ions citrate à 16 000 g pendant 1 heure, puis le culot est récupéré.

Le couplage des nanoparticules greffées en surface par du citrate avec la streptavidine est opéré suivant le protocole suivant :

**1.** Préparer fraichement une solution mixte de EDC [1]/Sulfo-NHS [2] (concentration 30 et 30 mg/mL, respectivement) dans du tampon MES [3] (pH 5-6).

**2.** Disperser par sonification (bain ultrasons) le culot des NPs dans 250 µL de la solution préparée en étape 1. Les pertes lors des centrifugations étant faibles, la concentration en ions vanadate reste de l'ordre de 5 mM, ce qui donne une concentration en nanoparticules de 48 nM. (La concentration en vanadate des solutions de nanoparticules a été déterminée par dissolution des particules dans un milieu acide suivie d'une détermination colorimétrique de la concentration en ions vanadate, telle que décrite dans la référence Abdesselem et al., ACS Nano 8, 11126-11137 (2014). La concentration molaire en nanoparticules a été déterminée à partir de la concentration en ions vanadate, comme décrit dans la référence Casanova et al., Appl. Phys. Lett 89, 253103 (2006)).

**3.** Préparer une solution de streptavidine (SA) de 100 nM dans du tampon phosphate pH 7,4 avec NaCl à 10 mM. Diluer la solution de streptavidine jusqu'à atteindre une concentration déterminée par le nombre de protéines greffées par nanoparticule souhaité (pour un rapport streptavidine : NPs de 1:1, 5:1 et 10:1, choisir, respectivement des concentrations de 4,8 nM, 24 nM et 48 nM). Rajouter 250 µL de cette solution à la solution de nanoparticules.

**4.** Laisser incuber entre 2 et 4h à température ambiante.

**5.** Rajouter 1 mL de PBST [4] et vortexer.

**6.** Faire une centrifugation de 6 500 g pendant 30 min et récupérer le culot pour éliminer les protéines non couplées aux NPs. Enlever totalement le surnageant. Rediserser les NPs couplées aux protéines dans 1mL de PBST et sonifier au bain à ultrasons. Répéter cette étape deux fois.

**7.** Récupérer les NPs couplées aux protéines dans 250 µL de PBS [5] avec 1 % de BSA [6].

**8.** Garder à 4°C pour utilisation immédiate ou aliquoter et garder à -80°C.

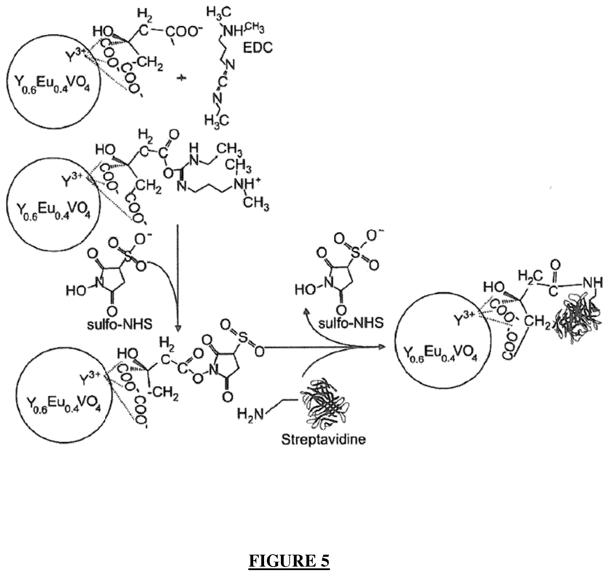

Le couplage des nanoparticules avec la streptavidine est représenté schématiquement en figure 5.

Matériel mis en œuvre pour l'essai de fonctionnalisation :

[1] *N*-(3-Dimethylaminopropyl)-*N'*-ethylcarbodiimide hydrochloride (EDC) (sigma, cat# E1769).
[2] *N*-Hydroxysulfosuccinimide sodium salt (Sulfo-NHS) (sigma, cat# 56485).
[3] 2-(*N*-morpholino)ethanesulfonic acid (MES) (10mM, pH 5-6).
[4] Tampon phosphate salin pH 7,4 (10 mM NaCl)+ 0,05% Tween 20 (PBST).
[5] Albumine de sérum de boeuf (BSA) (sigma, cat# A3059).
[6] Tampon phosphate salin (PBS) (pH 7,4, 10 mM NaCl).

Le nombre de molécules de streptavidine (SA) par nanoparticule (NP), noté R, caractérisé à l'issue du protocole de couplage, est déterminé *via* le dosage de la streptavidine par la méthode dite de BCA, selon le protocole détaillé ci-dessus.

*Protocole de caractérisation du rapport streptavidine : nanoparticules (SA :NPs)*

*i. Dosage de la SA par la méthode dite de BCA*

Principe

En milieu alcalin, les protéines réduisent $Cu^{2+}$ en Cu. Le sel de l'acide bicinchoninique (BCA) forme un complexe coloré avec les ions $Cu^{2+}$. Ce complexe est quantifiable grâce à son absorption à 562 nm.

Mode opératoire

Kit de test BCA de chez ThermoFisher (Pierce™ BCA Protein Assay Kit Cat. Num. 23225)
• Préparation du réactif du test $Cu^{2+}$/BCA selon le protocole du kit ThermoFisher.
• Préparation de la SA pour la courbe d'étalonnage. Pour la courbe de calibration, le test est réalisé trois fois.

TABLEAU 2 : concentration en streptavidine des solutions d'étalonnage

| Tubes | Concentration finale en SA ($\mu$g/mL) |
|-------|----------------------------------------|
| A | 2000 |
| B | 1500 |
| C | 1000 |
| D | 750 |
| E | 500 |
| F | 250 |
| G | 125 |
| H | 25 |
| I | 0 = blanc |

Le mode opératoire est le suivant :

- Prendre une plaque de 96 puits. Mettre 25 $\mu$L de chaque tube d'étalon A à I (de concentration connue en SA) ou de protéines conjuguées aux nanoparticules à doser dans les puits correspondants.
- Rajouter 200 $\mu$L du réactif du test $Cu^{2+}$/BCA dans chaque puits. Homogénéiser, couvrir et laisser incuber 30 min à 37°C.
- Lire les absorbances (A) à 562 nm en fonction de la concentration finale en tenant compte de la dilution. Etablir la relation d'étalonnage A= f (Concentration SA en $\mu$g/mL) par régression linéaire.

La concentration des protéines conjuguées aux nanoparticules à doser est déduite à partir de l'équation de la régression linéaire obtenue avec la courbe d'étalonnage.

*ii. Caractérisation du rapport SA : NPs*

La concentration massique de la streptavidine obtenue avec le test de BCA est convertie en concentration molaire via la formule suivante:

$$[SA]_{molaire}(moles/L) = \frac{[SA]massique\ (g/L)}{Masse\ Molaire\ (g/mole)}$$

Pour obtenir le rapport (R) du nombre de SA : NPs, nous appliquons enfin l'équation suivante :

$$R = \frac{[SA]\,molaire}{[NPs]\,molaire}$$

Le tableau 3 ci-dessous rassemble les valeurs obtenues pour les différents rapports de concentration entre la solution de streptavidine et la solution de nanoparticules de départ.

| Rapports de concentration entre la solution de streptavidine et la solution de nanoparticules | 1 :1 | 5 :1 | 10 :1 |
|---|---|---|---|
| Nombre déterminé de molécules de streptavidine par nanoparticule à l'issue du couplage | 0,97 | 3,8 | 9,29 |

TABLEAU 3 : Caractérisation du couplage nanoparticule-streptavidine pour différents rapports de concentration entre la solution de streptavidine et la solution de nanoparticules.

Comme il ressort des résultats présentés dans le tableau 3, le nombre de molécules de streptavidine par nanoparticule après couplage, est du même ordre que le rapport de concentration dans les solutions initiales, et indique ainsi un couplage de très bonne efficacité.

Les nanoparticules ainsi couplées à la streptavidine peuvent être avantageusement utilisées dans une méthode de diagnostic *in vitro* en couplant les nanoparticules à un anticorps biotinylé, comme illustré de manière schématique en figure 6.

Alternativement, il est également possible de coupler directement des anticorps aux nanoparticules greffées avec des ions citrate. Dans ce cas, le même protocole que ci-dessus est à utiliser en remplaçant la solution de streptavidine par une solution d'anticorps.

## EXEMPLE 5 (hors invention)

## Synthèse de nanoparticules $Y_{0,6}Eu_{0,4}VO_4$ à partir de métavanadate de sodium, avec ajout des ions tétraalkylammonium en fin de synthèse

Une suspension colloïdale de nanoparticules $Y_{0,6}Eu_{0,4}VO_4$ est préparée à partir de sels de sodium suivant la synthèse de l'exemple 2.

La purification est réalisée par dialyse.

On ajoute 1 équivalent (par rapport aux ions vanadate) d'hydroxyde de tétraméthylammonium $N(CH_3)_4OH$, on laisse 20 minutes, puis on dialyse à nouveau pour éliminer l'excédent de contre-ions jusqu'à atteindre une conductivité strictement inférieure à 100 $\mu$S.cm$^{-1}$.

Le potentiel zêta, déterminé comme décrit en exemple 1, au pH de la suspension obtenue de 8,9 est de -2,20 mV.

Il n'est pas possible d'obtenir le même résultat que celui obtenu avec le procédé de l'invention, en utilisant un procédé de synthèse des nanoparticules standard puis en ajoutant, à l'issue de la synthèse, des ions volumineux de type tétraalkylammonium.

Références

[1] « Bioconjugate Techniques », GT Hermanson, Academic Press, 1996 ;
[2] « Fluorescent and Luminescent Probes for Biological Activity. A Practical Guide to Technology for Quantitative Real-Time Analysis », Second Edition, Mason WT, Ed., Academic Press, 1999 ;
[3] Nam et al., Science 301, 1884-1886 (2003) ;
[4] Riwotzki et al., J. Phys. Chem. B 1998, 105, 12709-127 ;
[5] Huignard et al., Chem. Mater. 2000, 12, 1090-1094 ;
[6] Riwotzki et al., Angew. Chem. 2001, 40(3), 573-576 ;
[7] Lehmann et al., J. Am. Chem. Soc. 2004, 126(45), 14935-14942 ;
[8] Riwotzki et al., J. Phys.Chem. B.I, IB., 2000, 104, 2824-2828 ;
[9] Huignard et al., Chem. Mater. 2000, 12, 10920-1094 ;
[10] Giaume et al., Langmuir 2008, 24, 11018-11026 ;
[11] Huignard et al., Chem. Mater. 2002, 14, 2264-2269 ;
[12] Riwotzki et al., J. Phys. Chem. B 1998, 102, 10129-10135 ;

[13] Mialon et al., Acs Nano, 2008 - ACS Publication, vol. 2, No. 12, 2505-2512 ;

[14] Li et al., J. Phys. Chem. C 2008, 112, 6228-6231 ;

[15] Wu et al., J. Mater. Chem., 2003, 13, 1223-1228 ;

[16] Liang et al., J. Alloys and Compounds 552 (2013) 289-293 ;

[17] Wu et al., J. Phys. Chem. B 2006, 110, 15791-15796 ;

[18] Xu et al., Inorg. Chem. 2010, 49, 6706-6715 ;

[19] Takeshita et al., Journal of Luminescence 128 (2008) 1515-1522.

**Revendications**

1.  Particule luminescente comprenant une nanoparticule de formule :

    $$A_{1-x}Ln_xVO_{4(1-y)}(PO_4)_y \qquad (I)$$

    dans laquelle :

    . A est choisi parmi l'yttrium (Y), le gadolinium (Gd), le lanthane (La) et leurs mélanges ;
    . Ln est choisi parmi l'europium (Eu), le dysprosium (Dy), le samarium (Sm), le néodyme (Nd), l'erbium (Er), l'ytterbium (Yb) et leurs mélanges ;
    . $0 < x < 1$ ; et
    . $0 \leq y < 1$ ;

    **caractérisée en ce que** la nanoparticule présente en surface des cations tétraalkylammonium en une quantité telle que ladite nanoparticule présente un potentiel zêta, noté $\zeta$, inférieur ou égal à -28 mV, dans un milieu aqueux de pH $\geq$ 5, en particulier de pH $\geq$ 5,5, et de conductivité ionique strictement inférieure à 100 $\mu$S.cm$^{-1}$

2.  Particule selon la revendication précédente, **caractérisée en ce que** lesdits cations tétraalkylammonium sont associés à la nanoparticule *via* des interactions électrostatiques avec les ions de surface de la nanoparticule chargés négativement $O^{2-}$.

3.  Particule selon la revendication 1 ou 2, **caractérisée en ce que** lesdits cations tétraalkylammonium sont des cations de formule $NR_4^+$ avec R, identiques ou différents, représentant un groupe $C_1$-$C_6$-alkyl, en particulier $C_1$-$C_4$-alkyl et plus particulièrement $C_1$-$C_3$-alkyl ; de préférence lesdits cations tétraalkylammonium sont choisis parmi le tétraméthylammonium, le tétraéthylammonium, le tétrapropylammonium, le tétrabutylammonium et leurs mélanges, en particulier lesdits cations tétraalkylammonium sont des cations tétraméthylammonium.

4.  Particule selon l'une quelconque des revendications précédentes, **caractérisée en ce que** ladite nanoparticule présente un potentiel zêta $\zeta$ inférieur ou égal à -30 mV, dans un milieu aqueux de pH $\geq$ 6,5, en particulier de pH $\geq$ 7, et de conductivité ionique strictement inférieure à 100 $\mu$S.cm$^{-1}$.

5.  Particule selon l'une quelconque des revendications précédentes, ladite nanoparticule étant de formule (I')

    $$A_{1-x}L_{nx}VO_{4(1-y)}(PO_4)_y \cdot (NR_4^+)_z \qquad (I')$$

    dans laquelle :

    . A est choisi parmi Y, Gd, La et leurs mélanges ;
    . Ln est choisi parmi Eu, Dy, Sm, Nd, Er, Yb et leurs mélanges ;
    . $0 < x < 1$ ;
    . $0 \leq y < 1$ ;
    . R, identiques ou différents, représentent un groupe $C_1$-$C_6$-alkyl, en particulier $C_1$-$C_4$-alkyl, notamment $C_1$-$C_3$-alkyl, et plus particulièrement méthyle ; et
    . z représente le nombre de cations tétraalkylammonium $NR_4^+$ localisés à la surface de ladite nanoparticule, en particulier z est compris entre 100 et 10000.

6.  Particule selon l'une quelconque des revendications précédentes, dans laquelle :

y vaut 0 ; et/ou

A représente Y ; et/ou

Ln représente Eu.

**7.** Particule selon l'une quelconque des revendications précédentes, **caractérisée en ce que** ladite nanoparticule est de formule $Y_{1-x}Eu_xVO_4 \cdot (NR_4^+)_z$ (III'), dans laquelle x, R et z sont tels que définis en revendication 5, en particulier de formule $Y_{0,6}Eu_{0,4}(VO_4) \cdot (NR_4^+)_z$ (III'), dans laquelle z est tel que défini en revendication 5.

**8.** Particule selon l'une quelconque des revendications précédentes, **caractérisée en ce que** la nanoparticule de formule (I) est de forme ellipsoïdale allongée, en particulier présentant une longueur du grand axe, *a*, comprise entre 20 et 60 nm et une longueur du petit axe, b, comprise entre 10 et 30 nm.

**9.** Particule selon l'une quelconque des revendications précédentes, dans laquelle le produit entre le taux de dopage, x, en ions Ln, en particulier en Eu, et le rendement quantique de l'émission par la nanoparticule est maximisé, en particulier dans laquelle la nanoparticule présente une cristallinité imparfaite.

**10.** Particule selon l'une quelconque des revendications précédentes, **caractérisée en ce que** ladite nanoparticule est couplée à au moins un agent de ciblage.

**11.** Procédé de préparation de particules luminescentes comprenant une nanoparticule de formule :

$$A_{1-x}Ln_xVO_{4(1-y)}(PO_4)_y \qquad (I)$$

dans laquelle :

. A est choisi parmi l'yttrium (Y), le gadolinium (Gd), le lanthane (La) et leurs mélanges ;
. Ln est choisi parmi l'europium (Eu), le dysprosium (Dy), le samarium (Sm), le néodyme (Nd), l'erbium (Er), l'ytterbium (Yb) et leurs mélanges ;
. $0 < x < 1$; et
. $0 \leq y < 1$ ;

par réaction de co-précipitation, en milieu aqueux, à partir de précurseurs desdits éléments A et Ln, et en présence d'ions orthovanadate ($VO_4^{3-}$) et éventuellement phosphate ($PO_4^{3-}$) ;
ladite réaction étant opérée en présence d'une quantité efficace de cations tétraalkylammonium telle que ladite nanoparticule présente un potentiel zêta, noté $\zeta$, inférieur ou égal à -28 mV, dans un milieu aqueux de pH $\geq$ 5, en particulier de pH $\geq$ 5,5, et de conductivité ionique strictement inférieure à 100 $\mu$S.cm$^{-1}$.

**12.** Procédé selon la revendication précédente, **caractérisé en ce qu'**il comprend au moins les étapes consistant en :

(i) disposer d'une solution aqueuse, notée solution (1), comprenant des ions orthovanadate ($VO_4^{3-}$), et éventuellement des ions phosphates ($PO_4^{3-}$), et des cations tétraalkylammonium ;
(ii) ajouter à la solution aqueuse (1), une solution aqueuse, dite solution (2), comprenant lesdits précurseurs des éléments A et Ln, en particulier sous forme de sels, notamment de nitrates, dans des conditions propices à la formation par co-précipitation des nanoparticules de formule (I) ; et
(iii) récupérer les nanoparticules de formule (I) à la surface desquelles sont localisés des cations tétraalkylammonium, formées à l'issue de l'étape (ii).

**13.** Procédé selon la revendication 11 ou 12, dans lequel les cations tétraalkylammonium sont choisis parmi le tétraméthylammonium, le tétraéthylammonium, le tétrapropylammonium, le tétrabutylammonium et leurs mélanges, en particulier lesdits cations tétraalkylammonium sont des cations tétraméthylammonium.

**14.** Procédé selon l'une quelconque des revendications 11 à 13 pour la préparation de particules telles que définies selon l'une quelconque des revendications 5 à 9.

**15.** Procédé selon l'une quelconque des revendications 11 à 14, dans lequel les ions orthovanadate ($VO_4^{3-}$) sont générés *in situ* par réaction d'un sel de métavanadate, en particulier du métavanadate d'ammonium ($NH_4VO_3$), avec une base, en particulier une base source de cations tétraalkylammonium, et plus particulièrement avec un hydroxyde de tétraalkylammonium.

**16.** Procédé selon l'une quelconque des revendications 11 à 15, **caractérisé en ce qu'**il comprend en outre une ou plusieurs étapes de couplage des particules avec un ou plusieurs agents de ciblage.

**17.** Suspension aqueuse colloïdale comprenant des particules, telles que définies selon l'une quelconque des revendications 1 à 10, ou obtenues selon le procédé défini selon l'une quelconque des revendications 11 à 16.

**18.** Utilisation de particules telles que définies selon l'une quelconque des revendications 1 à 10 ou obtenues selon le procédé défini selon l'une quelconque des revendications 11 à 16, ou d'une suspension aqueuse colloïdale de ces particules, à titre d'agent de diagnostic.

**19.** Kit de diagnostic, comprenant au moins des particules telles que définies selon l'une quelconque des revendications 1 à 10 ou telles qu'obtenues selon le procédé défini selon l'une quelconque des revendications 11 à 16, ou une suspension aqueuse colloïdale de ces particules.

**Patentansprüche**

**1.** Lumineszierendes Partikel, umfassend ein Nanopartikel der Formel:

$$A_{1-x}Ln_xVO_{4(1-y)}(PO_4)_y \qquad (I)$$

wobei:

. A aus Yttrium (Y), Gadolinium (Gd), Lanthan (La) und Mischungen davon ausgewählt ist;
. Ln aus Europium (Eu), Dysprosium (Dy), Samarium (Sm), Neodym (Nd), Erbium (Er), Ytterbium (Yb) und Mischungen davon ausgewählt ist;
. $0 < x < 1$; und
. $0 \leq y < 1$ ;

**dadurch gekennzeichnet, dass** das Nanopartikel an der Oberfläche Tetraalkylammoniumkationen in einer solchen Menge aufweist, dass das Nanopartikel ein Zeta-Potential, das als $\zeta$ bezeichnet wird, kleiner als oder gleich -28 mV in einem wässrigen Medium mit pH $\geq$ 5, insbesondere mit pH $\geq$ 5,5, und mit einer Ionenleitfähigkeit von streng weniger als 100 $\mu$S.cm$^{-1}$ aufweist.

**2.** Partikel nach dem vorhergehenden Anspruch, **dadurch gekennzeichnet, dass** die Tetraalkylammoniumkationen über elektrostatische Wechselwirkungen mit den negativ geladenen $O^{2-}$-Oberflächenionen des Nanopartikels mit dem Nanopartikel assoziiert sind.

**3.** Partikel nach Anspruch 1 oder 2, **dadurch gekennzeichnet, dass** es sich bei den Tetraalkylammoniumkationen um Kationen der Formel $NR_4^+$ handelt, wobei die Reste R gleich oder verschieden sind und für eine $C_1$-$C_6$-Alkylgruppe, insbesondere eine $C_1$-$C_4$-Alkylgruppe und spezieller eine $C_1$-$C_3$-Alkylgruppe stehen; vorzugsweise die Tetraalkylammoniumkationen aus Tetramethylammonium, Tetraethylammonium, Tetrapropylammonium, Tetrabutylammonium und Mischungen davon ausgewählt sind und es sich bei den Tetraalkylammoniumkationen insbesondere um Tetramethylammoniumkationen handelt.

**4.** Partikel nach einem der vorhergehenden Ansprüche, **dadurch gekennzeichnet, dass** das Nanopartikel ein Zeta-Potential $\zeta$ kleiner als oder gleich -30 mV in einem wässrigen Medium mit pH $\geq$ 6,5, insbesondere mit pH $\geq$ 7, und mit einer Ionenleitfähigkeit von streng weniger als 100 $\mu$S.cm$^{-1}$ aufweist.

**5.** Partikel nach einem der vorhergehenden Ansprüche, wobei das Nanopartikel die Formel (I') aufweist:

$$A_{1-x}Ln_xVO_{4(1-y)}(PO_4)_y \cdot (NR_4^+)_z \qquad (I')$$

wobei

. A aus Y, Gd, La und Mischungen davon ausgewählt ist;
. Ln aus Eu, Dy, Sm, Nd, Er, Yb und Mischungen davon ausgewählt ist;
. $0 < x < 1$; und

. $0 \leq y < 1$;

. die Reste R gleich oder verschieden sind und für eine $C_1$-$C_6$-Alkylgruppe, insbesondere eine $C_1$-$C_4$-Alkylgruppe, speziell eine $C_1$-$C_3$-Alkylgruppe und spezieller eine Methylgruppe stehen; und

. z für die Zahl der Tetraalkylammoniumkationen $NR_4^+$ an der Oberfläche des Nanopartikels steht, wobei z insbesondere zwischen 100 und 10.000 liegt.

6. Partikel nach einem der vorhergehenden Ansprüche, wobei:

   y gleich 0 ist; und/oder
   A für Y steht; und/oder
   Ln für Eu steht.

7. Partikel nach einem der vorhergehenden Ansprüche, **dadurch gekennzeichnet, dass** das Nanopartikel die Formel $Y_{1-x}Eu_xVO_4 \cdot (NR_4^+)_z$ (III'), wobei x, R und z wie in Anspruch 5 definiert sind, insbesondere die Formel $Y_{0,6}Eu_{0,4}(VO_4) \cdot (NR_4^+)_z$ (III''), wobei z wie in Anspruch 5 definiert ist, aufweist.

8. Partikel nach einem der vorhergehenden Ansprüche, **dadurch gekennzeichnet, dass** das Nanopartikel der Formel (I) eine lang gestreckte ellipsoide Form aufweist und insbesondere eine Länge der Hauptachse a zwischen 20 und 60 nm und eine Länge der Nebenachse *b* zwischen 10 und 30 nm aufweist.

9. Partikel nach einem der vorhergehenden Ansprüche, wobei das Produkt des Dotierungsgrads x mit Ln-Ionen, insbesondere mit Eu, und der Quantenausbeute der Emission des Nanopartikels maximiert ist, insbesondere wobei das Nanopartikel eine unvollkommene Kristallinität aufweist.

10. Partikel nach einem der vorhergehenden Ansprüche, **dadurch gekennzeichnet, dass** das Nanopartikel mit mindestens einem Zielsteuerungsmittel gekoppelt ist.

11. Verfahren zur Herstellung von lumineszierenden Partikeln, umfassend ein Nanopartikel der Formel:

$$A_{1-x}Ln_xVO_{4(1-y)}(PO_4)_y \qquad (I)$$

wobei:

   . A aus Yttrium (Y), Gadolinium (Gd), Lanthan (La) und Mischungen davon ausgewählt ist;
   . Ln aus Europium (Eu), Dysprosium (Dy), Samarium (Sm), Neodym (Nd), Erbium (Er), Ytterbium (Yb) und Mischungen davon ausgewählt ist;
   . $0 < x < 1$; und
   . $0 \leq y < 1$ ;

durch eine Copräzipitationsreaktion in wässrigem Medium aus Vorstufen der Elemente A und Ln und in Gegenwart von Orthovanadat $(VO_4^{3-})$- und gegebenenfalls Phosphat $(PO_4^{3-})$-Ionen; wobei die Reaktion in Gegenwart einer wirksamen Menge von Tetraalkylammoniumkationen durchgeführt wird, so dass das Nanopartikel ein Zeta-Potential, das als $\zeta$ bezeichnet wird, kleiner als oder gleich -28 mV in einem wässrigen Medium mit pH $\geq$ 5, insbesondere mit pH $\geq$ 5,5, und mit einer Ionenleitfähigkeit von streng weniger als 100 $\mu$S.cm$^{-1}$ aufweist.

12. Verfahren nach dem vorhergehenden Anspruch, **dadurch gekennzeichnet, dass** es mindestens folgende Schritte umfasst:

   (i) Bereitstellen einer wässrigen Lösung, die als Lösung (1) bezeichnet wird, umfassend Orthovanadat $(VO_4^{3-})$- und gegebenenfalls Phosphat $(PO_4^{3-})$-Ionen und Tetraalkylammoniumkationen;
   (ii) Zugeben einer wässrigen Lösung, die als Lösung (2) bezeichnet wird, umfassend die Vorstufen der Elemente A und Ln, insbesondere in Form von Salzen, speziell von Nitraten, zu der wässrigen Lösung (1) unter Bedingungen, die für die Bildung der Nanopartikel der Formel (I) durch Copräzipitation günstig sind; und
   (iii) Gewinnen der am Ende von Schritt (ii) gebildeten Nanopartikel der Formel (I), an deren Oberfläche sich Tetraalkylammoniumkationen befinden.

13. Verfahren nach Anspruch 11 oder 12, wobei die Tetraalkylammoniumkationen aus Tetramethylammonium, Tetraethylammonium, Tetrapropylammonium, Tetrabutylammonium und Mischungen davon ausgewählt werden und es sich bei den Tetraalkylammoniumkationen insbesondere um Tetramethylammoniumkationen handelt.

14. Verfahren nach einem der Ansprüche 11 bis 13 zur Herstellung von Partikeln gemäß einem der Ansprüche 5 bis 9.

15. Verfahren nach einem der Ansprüche 11 bis 14, wobei die Orthovanadat ($VO_4^{3-}$)-Ionen in situ durch Reaktion eines Metavanadatsalzes, insbesondere Ammoniummetavanadat ($NH_4VO_3$) mit einer Base, insbesondere einer Base, bei der es sich um eine Quelle von Tetraalkylammoniumkationen handelt, und spezieller mit einem Tetraalkylammoniumhydroxid, in situ erzeugt werden.

16. Verfahren nach einem der Ansprüche 11 bis 15, **dadurch gekennzeichnet, dass** es außerdem einen oder mehrere Schritte des Koppelns der Partikel mit einem oder mehreren Zielsteuerungsmitteln umfasst.

17. Kolloidale wässrige Suspension, umfassend Partikel gemäß einem der Ansprüche 1 bis 10 oder nach dem Verfahren gemäß einem der Ansprüche 11 bis 16 erhaltene Partikel.

18. Verwendung von Partikeln gemäß einem der Ansprüche 1 bis 10 oder nach dem Verfahren gemäß einem der Ansprüche 11 bis 16 erhaltenen Partikeln oder einer kolloidalen wässrigen Suspension dieser Partikel als Diagnostika.

19. Diagnosekit, umfassend zumindest Partikel gemäß einem der Ansprüche 1 bis 10 oder nach dem Verfahren gemäß einem der Ansprüche 11 bis 16 erhaltene Partikel oder eine kolloidale wässrige Suspension dieser Partikel.

**Claims**

1. A luminescent particle comprising a nanoparticle of formula:

$$A_{1-x}Ln_xVO_{4(1-y)}(PO_4)_y \qquad (I)$$

in which:

. A is selected from yttrium (Y), gadolinium (Gd), lanthanum (La), and mixtures thereof;
. Ln is selected from europium (Eu), dysprosium (Dy), samarium (Sm), neodymium (Nd), erbium (Er), ytterbium (Yb), and mixtures thereof;
. $0 < x < 1$; and
. $0 \leq y < 1$;

**characterized in that** the nanoparticle has, at the surface, tetraalkylammonium cations in an amount such that said nanoparticle has a zeta potential, $\zeta$, of less than or equal to -28 mV in an aqueous medium with a pH $\geq$ 5, more particularly with a pH $\geq$ 5.5, and with an ionic conductivity of strictly less than 100 $\mu$S.cm$^{-1}$.

2. The particle as claimed in the preceding claim, **characterized in that** said tetraalkylammonium cations are associated with the nanoparticle *via* electrostatic interactions with the negatively charged $O^{2-}$ surface ions of the nanoparticle.

3. The particle as claimed in claim 1 or 2, **characterized in that** said tetraalkylammonium cations are cations of formula $NR_4^+$ where R, which are identical or different, represent a $C_1$-$C_6$-alkyl, more particularly $C_1$-$C_4$-alkyl and more particularly $C_1$-$C_3$-alkyl group; preferably said tetraalkylammonium cations are selected from tetramethylammonium, tetraethylammonium, tetrapropylammonium, tetrabutylammonium, and mixtures thereof, said tetraalkylammonium cations more particularly being tetramethylammonium cations.

4. The particle as claimed in any of the preceding claims, **characterized in that** said nanoparticle has a zeta potential $\zeta$ of less than or equal to -30 mV in an aqueous medium with a pH $\geq$ 6.5, more particularly with a pH $\geq$ 7, and with an ionic conductivity of strictly less than 100 $\mu$S.cm$^{-1}$.

5. The particle as claimed in any of the preceding claims, said nanoparticle being of formula (I')

$$A_{1-x}Ln_xVO_{4(1-y)}(PO_4)_y \cdot (NR_4^+)_z \qquad (I')$$

in which:

. A is selected from Y, Gd, La, and mixtures thereof;
. Ln is selected from Eu, Dy, Sm, Nd, Er, Yb, and mixtures thereof;
. $0 < x < 1$;
. $0 \leq y < 1$;
. R, which are identical or different, represent a $C_1$-$C_6$-alkyl, more particularly $C_1$-$C_4$-alkyl, especially $C_1$-$C_3$-alkyl, and very particularly methyl group; and
. z represents the number of tetraalkylammonium cations $NR_4^+$ located on the surface of said nanoparticle, z more particularly being between 100 and 10 000.

6. The particle as claimed in any of the preceding claims, wherein:

y is 0; and/or
A represents Y; and/or
Ln represents Eu.

7. The particle as claimed in any of the preceding claims, **characterized in that** said nanoparticle is of formula $Y_{1-x}Eu_xVO_4 . (NR_4^+)_z$ (III'), in which x, R, and z are as defined in claim 5, more particularly of formula $Y_{0.6}Eu_{0.4}(VO_4) . (NR_4^+)_z$ (III'), in which z is as defined in claim 5.

8. The particle as claimed in any of the preceding claims, **characterized in that** the nanoparticle of formula (I) has an elongated ellipsoidal shape, more particularly having a length of the major axis, *a*, of between 20 and 60 nm and a length of the minor axis, b, of between 10 and 30 nm.

9. The particle as claimed in any of the preceding claims, wherein the product of the degree of doping, x, with Ln ions, more particularly with Eu, and the quantum yield of the emission by the nanoparticle is maximized, more particularly wherein the nanoparticle exhibits an imperfect crystallinity.

10. The particle as claimed in any of the preceding claims, **characterized in that** said nanoparticle is coupled to at least one targeting agent.

11. A method for preparing luminescent particles comprising a nanoparticle of formula:

$$A_{1-x}Ln_xVO_{4(1-y)}(PO_4)_y \qquad (I)$$

in which:

. A is selected from yttrium (Y), gadolinium (Gd), lanthanum (La), and mixtures thereof;
. Ln is selected from europium (Eu), dysprosium (Dy), samarium (Sm), neodymium (Nd), erbium (Er), ytterbium (Yb), and mixtures thereof;
. $0 < x < 1$; and
. $0 \leq y < 1$;

by coprecipitation reaction, in aqueous medium, from precursors of said elements A and Ln, and in the presence of orthovanadate ($VO_4^{3-}$) and optionally phosphate ($PO_4^{3-}$) ions;
said reaction being performed in the presence of an effective amount of tetraalkylammonium cations such that said nanoparticle has a zeta potential, $\zeta$, of less than or equal to -28 mV in an aqueous medium with a pH $\geq 5$, more particularly with a pH $\geq 5.5$, and with an ionic conductivity of strictly less than 100 $\mu S.cm^{-1}$.

12. The method as claimed in the preceding claim, **characterized in that** it comprises at least the steps of:

(i) providing an aqueous solution, called solution (1), comprising orthovanadate ions ($VO_4^{3-}$), and optionally phosphate ions ($PO_4^{3-}$), and tetraalkylammonium cations;
(ii) admixing the aqueous solution (1) with an aqueous solution, called solution (2), comprising said precursors of the elements A and Ln, more particularly in the form of salts, especially nitrates, under conditions conducive to the formation by coprecipitation of the nanoparticles of formula (I); and

# EP 3 625 308 B1

(iii) recovering the nanoparticles of formula (I) with tetraalkylammonium cations located on their surface that are formed at the end of step (ii).

13. The method as claimed in claim 11 or 12, wherein the tetraalkylammonium cations are selected from tetramethylammonium, tetraethylammonium, tetrapropylammonium, tetrabutylammonium, and mixtures thereof, said tetraalkylammonium cations more particularly being tetramethylammonium cations.

14. The method as claimed in any of claims 11 to 13 for preparing particles as defined in any of claims 5 to 9.

15. The method as claimed in any of claims 11 to 14, wherein the orthovanadate ions ($VO_4^{3-}$) are generated *in situ* by reacting a metavanadate salt, more particularly ammonium metavanadate ($NH_4VO_3$), with a base, more particularly a base which is a source of tetraalkylammonium cations, and very particularly with a tetraalkylammonium hydroxide.

16. The method as claimed in any of claims 11 to 15, **characterized in that** it further comprises one or more steps of coupling the particles with one or more targeting agents.

17. A colloidal aqueous suspension comprising particles as defined in any of claims 1 to 10 or obtained by the method defined in any of claims 11 to 16.

18. The use of particles as defined in any of claims 1 to 10 or obtained by the method defined in any of claims 11 to 16, or of a colloidal aqueous suspension of these particles, as a diagnostic agent.

19. A diagnostic kit comprising at least particles as defined in any of claims 1 to 10 or as obtained by the method defined in any of claims 11 to 16, or a colloidal aqueous suspension of these particles.

Fig. 1a

Fig. 1b

**FIGURE 1**

**FIGURE 2**

**FIGURE 3**

**Fig.4a**                    **Fig.4b**

**FIGURE 4**

**FIGURE 5**

**FIGURE 6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1232226 A
- US 8337804 B
- EP 1282824 A
- WO 2016079069 A

**Littérature non-brevet citée dans la description**

- **GIAUME et al.** *Mater. Res. Soc. Symp. Proc.,* 2006, vol. 942
- **ABDESSELEM et al.** *ACS Nano,* 2014, vol. 8, 11126-11137
- **CASANOVA et al.** *Appl. Phys. Lett,* 2006, vol. 89, 253103
- **GT HERMANSON.** Bioconjugate Techniques. Academic Press, 1996
- Fluorescent and Luminescent Probes for Biological Activity. A Practical Guide to Technology for Quantitative Real-Time Analysis. Academic Press, 1999
- **NAM et al.** *Science,* 2003, vol. 301, 1884-1886
- **RIWOTZKI et al.** *J. Phys. Chem. B,* 1998, vol. 105, 12709-127
- **HUIGNARD et al.** *Chem. Mater.,* 2000, vol. 12, 1090-1094
- **RIWOTZKI et al.** *Angew. Chem.,* 2001, vol. 40 (3), 573-576
- **LEHMANN et al.** *J. Am. Chem. Soc.,* 2004, vol. 126 (45), 14935-14942
- **RIWOTZKI et al.** *J. Phys.Chem. B.I,* 2000, vol. 104, 2824-2828
- **HUIGNARD et al.** *Chem. Mater.,* 2000, vol. 12, 10920-1094
- **GIAUME et al.** *Langmuir,* 2008, vol. 24, 11018-11026
- **HUIGNARD et al.** *Chem. Mater.,* 2002, vol. 14, 2264-2269
- **RIWOTZKI et al.** *J. Phys. Chem. B,* 1998, vol. 102, 10129-10135
- **MIALON et al.** Acs Nano. ACS Publication, 2008, vol. 2, 2505-2512
- **LI et al.** *J. Phys. Chem. C,* 2008, vol. 112, 6228-6231
- **WU et al.** *J. Mater. Chem.,* 2003, vol. 13, 1223-1228
- **LIANG et al.** *J. Alloys and Compounds,* 2013, vol. 552, 289-293
- **WU et al.** *J. Phys. Chem. B,* 2006, vol. 110, 15791-15796
- **XU et al.** *Inorg. Chem.,* 2010, vol. 49, 6706-6715
- **TAKESHITA et al.** *Journal of Luminescence,* 2008, vol. 128, 1515-1522